(19) Europäisches Patentamt
European Patent Office
Office européen des brevets

(11) Veröffentlichungsnummer: **0 114 572**
**A1**

(12) # EUROPÄISCHE PATENTANMELDUNG

(21) Anmeldenummer: **83810595.5**

(22) Anmeldetag: **15.12.83**

(51) Int. Cl.³: **C 07 D 471/04**
A 61 K 31/44
//C07D213/36, C07D213/54,
C07D213/65, C07D213/84,
C07D213/89, (C07D471/04,
235/00, 221/00)

(30) Priorität: **21.12.82 US 451903**

(43) Veröffentlichungstag der Anmeldung:
**01.08.84 Patentblatt 84/31**

(84) Benannte Vertragsstaaten:
**AT BE CH DE FR GB IT LI LU NL SE**

(71) Anmelder: **CIBA-GEIGY AG**
**Postfach**
**CH-4002 Basel(CH)**

(72) Erfinder: **Browne, Leslie J., Dr.**
**41 Malapardis Road**
**Morris Plains, N.J. 07950(US)**

(54) **Substituierte Imidazo(1,5-a)pyridine.**

(57) Die vorliegende Erfindung betrifft Verbindungen der Formel I

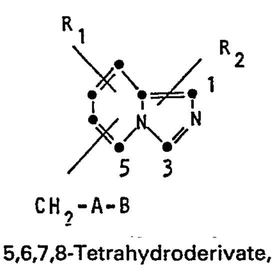

(I)

oder ihre 5,6,7,8-Tetrahydroderivate, worin $R_1$ für Wasserstoff, Halogen, Niederalkyl, Niederalkoxy, Hydroxy oder Aryl-niederalkoxy steht, $R_2$ Wasserstoff, Halogen oder Niederalkyl bedeutet, A für Alkylen mit 1 bis 12 Kohlenstoffatomen, Alkinylen oder Alkenylen mit 2 bis 12 Kohlenstoffatomen steht, und B 5-Tetrazolyl, Hydroxy-carbamoyl oder unsubstituiertes oder durch Niederalkyl substituiertes 4,5-Dihydro-2-oxazolyl bedeutet, und ihre Salze. Sie besitzen eine die Thromboxan-Synthetase hemmende Wirkung. Die Erfindung umfasst ferner Verfahren zur Herstellung dieser Verbindungen, ihre Verwendung, diese Verbindungen enthaltende pharmazeutische Präparate und Zwischenprodukte.

EP 0 114 572 A1

0114572

CIBA-GEIGY AG
Basel (Schweiz)

4-14240/+

## Substituierte Imidazo[1,5-a]pyridine

Die Erfindung betrifft neue Imidazo[1,5-a]pyridine und Verfahren zu Ihrer Herstellung.

Die Erfindung betrifft insbesondere Imidazo[1,5-a]pyridine der allgemeinen Formel I

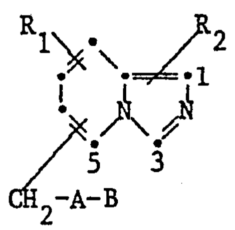

(I)

oder ihre 5,6,7,8-Tetrahydroderivate, worin $R_1$ für Wasserstoff, Halogen, Niederalkyl, Niederalkoxy, Hydroxy oder Aryl-niederalkoxy steht, $R_2$ Wasserstoff, Halogen oder Niederalkyl bedeutet, A für Alkylen mit 1 bis 12 Kohlenstoffatomen, Alkinylen oder Alkenylen mit 2 bis 12 Kohlenstoffatomen steht, und B 5-Tetrazolyl, Hydroxycarbamoyl oder unsubstituiertes oder durch Niederalkyl substituiertes 4,5-Dihydro-2-oxazolyl bedeutet, und ihre Salze, insbesondere ihre therapeutisch verwendbaren Salze, sowie Verfahren zu ihrer Herstellung, pharmazeutische Präparate enthaltend diese Verbindungen, sowie ihre therapeutische Verwendung.

Bevorzugt sind Verbindungen der allgemeinen Formel I, in welchen die Gruppe $-CH_2-A-B$ an die 5-Stellung gebunden ist. Sehr nützlich sind Verbindungen der Formel I, worin A Alkylen mit 1 bis 12 Kohlenstoffatomen bedeutet.

Besonders bevorzugt sind Verbindungen der allgemeinen Formel II

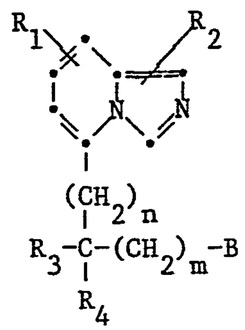

(II)

oder ihre 5,6,7,8-Tetrahydroderivate, worin jedes der Symbole $R_1$, $R_2$, $R_3$ und $R_4$ Wasserstoff oder Niederalkyl mit 1 bis 4 Kohlenstoffatomen bedeutet, n eine ganze Zahl von 1 bis 7 ist, m 0 oder 1 ist, B für 5-Tetrazolyl, Hydroxycarbamoyl oder unsubstituiertes oder durch Niederalkyl substituiertes 4,5-Dihydro-2-oxazolyl steht, und ihre Salze, insbesondere ihre therapeutisch verwendbaren Salze.

Besonders hervorzuheben sind Verbindungen der allgemeinen Formel II oder ihre 5,6,7,8-Tetrahydroderivate, worin jedes der Symbole $R_1$, $R_2$, $R_3$ und $R_4$ Wasserstoff, Methyl oder Aethyl bedeutet, $(CH_2)_n$ für Propylen, Butylen, Pentylen oder Hexylen steht, m 0 oder 1 ist und B 5-Tetrazolyl, Hydroxycarbamoyl oder 4,5-Dihydro-2-oxazolyl bedeutet, und ihre Salze, insbesondere ihre therapeutisch verwendbaren Salze.

Bevorzugt sind weiter Verbindungen der Formel II, in welchen die Gruppe

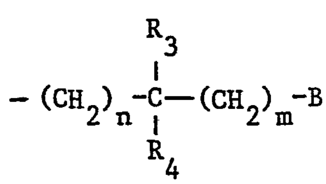

in der 5-Stellung angeknüpft ist.

Ueberaus nützlich sind Verbindungen der allgemeinen Formel III

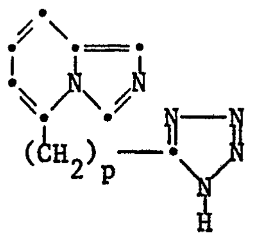

(III)

oder ihre 5,6,7,8-Tetrahydroderivate, worin p eine ganze Zahl von 3 bis 8 bedeutet, und ihre Salze, insbesondere ihre therapeutisch verwendbaren Salze.

Besonders wertvoll sind Verbindungen der allgemeinen Formel III oder ihre 5,6,7,8-Tetrahydroderivate, worin p 4,5 oder 6 bedeutet, und ihre Salze, insbesondere ihre therapeutisch verwendbaren Salze.

Die allgemeinen Definitionen, welche hier verwendet werden, haben innerhalb des Umfangs der vorliegenden Erfindung die folgenden Bedeutungen.

Ein Alkylenrest bedeutet Alkylen mit 1 bis 12 Kohlenstoffatomen, der geradkettig oder verzweigt sein kann, und vorzugsweise für Propylen, Butylen, Pentylen oder Hexylen steht, wobei die genannten Reste unsubstituiert oder durch eine oder mehrere Niederalkylgruppen substituiert sind, mit der Massgabe, dass die Summe der Kohlenstoffatome nicht grösser als 12 ist.

Der Ausdruck Alkenylen bedeutet einen Alkenylenrest mit 2 bis 12 Kohlenstoffatomen,wobei ein solcher Rest geradkettig oder verzweigt sein kann, und vorzugsweise für Propenylen, 1- oder 2-Butenylen, 1- oder 2-Pentenylen oder 1-, 2- oder 3-Hexenylen steht. Die genannten Reste sind unsubstituiert oder durch eine oder mehrere Niederalkylgruppen substituiert, mit der Massgabe, dass die Summe der Kohlenstoffatome 12 nicht übersteigt.

Der Begriff Alkinylen bezeichnet einen Alkinylrest mit 2 bis 12

Kohlenstoffatomen, der geradkettig oder verzweigt ist, und vorzugsweise für Propinylen, 1- oder 2-Butinylen, 1- oder 2-Pentinylen, 1-, 2- oder 3-Hexinylen steht. Diese Reste sind unsubstituiert oder durch eine oder mehrere Niederalkylgruppen substituiert, wobei die Summe der Kohlenstoffatome 12 nicht übersteigt.

Der Ausdruck "nieder" definiert in den oben oder nachfolgend genannten organischen Resten oder Verbindungen solche mit höchstens 7, vorzugsweise 4 und insbesondere 1 oder 2 Kohlenstoffatomen.

Eine Niederalkylgruppe enthält vorzugsweise 1 bis 4 Kohlenstoffatome und steht z.B. für Aethyl, Propyl, Butyl und insbesondere für Methyl.

Aryl, z.B. in einer Arylniederalkoxy-Gruppe, bedeutet vorzugweise unsubstituiertes oder durch Niederalkyl, Halogen oder Niederalkoxy mono- oder disubstituiertes Phenyl.

Eine Arylniederalkoxy-Gruppe ist insbesondere Benzyloxy.

Niederalkoxy enthält vorzugsweise 1 bis 4 Kohlenstoffatome und bedeutet z.B. Methoxy, Propoxy, Isopropoxy und insbesondere Aethoxy.

Halogen ist vorzugsweise Fluor und Chlor, kann aber auch Brom oder Jod bedeuten.

Salze sind vorzugsweise therapeutisch verwendbare Salze, z.B. Metall- oder Ammoniumsalze der genannten Verbindungen der Formel I, worin B 5-Tetrazolyl oder Hydroxycarbamoyl bedeutet, insbesondere Alkalimetall- oder Erdalkalimetallsalze, z.B. Natrium-, Kalium-, Magnesium- oder Calciumsalze; in erster Linie leicht kristallisierende Ammoniumsalze. Diese werden abgeleitet von Ammoniak oder organischen Aminen, z.B. Mono-, Di- oder Tri-nieder-(alkyl, cycloalkyl oder hydroxyalkyl)-aminen, Niederalkylendiaminen oder (Hydroxy-niederalkyl oder Arylniederalkyl)-niederalkylammonium-Basen, z.B. Methylamin, Diäthylamin, Triäthylamin, Dicyclohexylamin, Triäthanolamin, Aethylendiamin, Tris-(hydroxymethyl)-aminomethan oder Benzyl-trimethylammoniumhydroxid. Die genannten Verbindungen der Formel I bilden Säureadditionssalze. Diese werden vorzugsweise mit solchen Säuren, welche therapeutisch verwendbare Säureadditionssalze ergeben,hergestellt. Säuren, die therapeutisch verwendbare Säureadditionssalze ergeben, sind z.B. starke Mineralsäuren, wie Halogenwasserstoffsäuren, z.B. Chlorwasserstoff- oder Bromwasserstoffsäure, Schwefel-, Phosphor-, Salpeter- oder Perchlorsäure; oder organische Säuren, wie aliphatische oder aromatische Carbon- oder Sulfonsäuren, z.B. Ameisen-, Essig-, Propion-, Bernstein-, Glykol-, Milch-, Aepfel-, Wein-, Glucon-, Zitronen-, Ascorbin-, Malein-, Fumar-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-,Pamoe-, Nikotin-, Methansulfon-, Aethansulfon-, Hydroxyäthansulfon-, Benzolsulfon-, Toluolsulfon-, Naphthalinsulfon-, Sulfanil- oder Cyclohexylsulfaminsäure.

Die Verbindungen der Erfindung zeigen wertvolle pharmakologische Eigenschaften, z.B. kardiovaskuläre Effekte, durch selektive Hemmung der Thromboxan-Ausschüttung in Säugern. Diese Hemmung kommt durch selektive Verminderung der Thromboxan-Synthetase zustande. Die Verbindungen sind daher nützlich in der Behandlung von Krankheiten, welche auf Thromboxan-Synthetase-Hemmung bei Säugern, einschliesslich Menschen, ansprechen.

Diese Wirkungen können durch in vitro Versuche oder in vivo Tierversuche, vorzugsweise an Säugetieren, z.B. Meerschweinchen, Mäusen, Ratten, Katzen, Hunden oder Affen nachgewiesen werden. Die genannten Verbindungen können ihnen enteral oder parenteral, vorzugsweise oral oder subkutan, intravenös oder intraperitoneal, z.B. durch Gelatine-Kapseln oder in Form von Stärke enthaltenden Suspensionen oder wässerigen Lösungen, verabreicht werden. Die verwendete Dosis kann in einem Bereich von ungefähr zwischen 0,01 und 100 mg/kg/Tag, vorzugsweise ungefähr 0,05 und 50 mg/kg/Tag, insbesondere ungefähr 0,1 und 25 mg/kg/Tag liegen.

Die in vitro Hemmung des Thromboxan-Synthetase-Enzyms kann analog zu der Methode von Sun, Biochem. Biophys. Res. Comm. 74, 1432 (1977) nachgewiesen werden. Das Testverfahren wird wie folgt durchgeführt: $^{14}C$-Arachidonsäure wird mit einem Enzymgemisch-Präparat, bestehend aus solubilisierter und partiell gereinigter Prostaglandin-cyclo-oxygenase von Schaf-Samenblasen und aus einem rohen Mikrosomen-Präparat von Thromboxan-Synthetase von lysierten menschlichen Blutplättchen, inkubiert. Die Testverbindung (gelöst in einem Puffer, oder falls nötig, in wenig Aethanol) wird zu dem Inkubationsmedium gegeben. Am Ende der Inkubationsperiode (30 Minuten) wird das Prostaglandin $E_2$ ($PGE_2$) durch Hinzufügen von Natriumborhydrid zu einem Gemisch von Prostaglandin $F_2\alpha$ und $F_2\beta$ [$PGF_2(\alpha+\beta)$] reduziert. Die radioaktiven Produkte und das überschüssige Substrat werden mit Essigsäureäthylester extrahiert und der Extrakt wird zur Trockene eingedampft. Der Rückstand wird in Aceton gelöst, auf Dünnschichtplatten tropfenweise aufgetragen und mit einem Lösungsmittelsystem von Toluol: Aceton:Eisessig [100:100:3 (Volumen)] chromatographiert. Die radioaktiven Zonen werden lokalisiert. Die Zonen von Thromboxan $B_2$ ($TxB_2$) und $PGF_2\alpha+\beta$ werden in Szintillationsröhrchen für Flüssigkeiten übertragen und gezählt. Der Quotient der Zahlenwerte von $TxB_2/PGF_2\alpha+\beta$ wird für jede Konzentration der Testverbindung berechnet und die $IC_{50}$-Werte werden graphisch ermittelt. Dieser Wert ist die Konzentration der Testverbindung, bei welcher der Quotient von $TxB_2/PGF_2\alpha+\beta$ auf 50% des Kontrollwertes reduziert wird.

Die in vitro Wirkung auf Prostaglandin-cyclo-oxygenase wird gemäss einer Modifikation der Methode von Takeguchi et al, welche in Biochemistry 10, 2372 (1971) beschrieben ist, gemessen. Das Testverfahren ist wie folgt:

Lyophiliserte Samenblasen-Mikrosomen von Schafen werden als Enzympräparat für die Prostaglandin-Synthese verwendet. Es wird die Umwandlung von $^{14}C$-Arachidonsäure in $PGE_2$ gemessen. Die Testverbindungen (gelöst in einem Puffer, oder wenn nötig in wenig Aethanol) werden zu dem Inkubationsgemisch gegeben. Die Prostaglandine werden extrahiert und durch Dünnschichtchromatographie aufgetrennt. Die Platten werden überprüft, die dem $PGE_2$ entsprechenden radioaktiven Zonen werden in Szintillationsröhrchen für Flüssigkeiten übertragen und ihre Radioaktivität gezählt. Die $IC_{50}$-Werte der Hemmung werden graphisch ermittelt. Dieser Wert bedeutet die Konzentration der Testverbindung, welche die Menge des synthetisierten $PGE_2$ um 50% reduziert.

Die in-vitro Wirkung auf Prostacyclin-($PGI_2$)-Synthetase wird analog zu der Methode von Sun et al., Prostaglandins 14, 1055 (1977) gemessen. Das Testverfahren ist wie folgt:

$^{14}C$-Arachidonsäure wird mit einem Enzymgemisch, bestehend aus solubilisierter und partiell gereinigter Prostaglandin-cyclo-oxygenase von Schaf-Samenblasen und aus rohem $PGI_2$-Synthetase in der Form einer Mikrosomen-Fraktion von Aorten von Rindern, inkubiert.

Die Testverbindung (gelöst in einem Puffer, oder wenn nötig, in wenig Aethanol) wird in das Inkubationsmedium gegeben. Das Reaktionsgemisch wird in 100 mMolarem Tris HCl (pH 7,5) 30 Minuten bei 37° inkubiert, auf den pH-Wert 3 angesäuert und mit Essigsäureäthylester extrahiert. Der Extrakt wird zur Trockene eingedampft, der Rückstand in Aceton gelöst auf Dünnschicht-Platten aufgetragen und mit einem von Sun et al. beschriebenen Lösungsmittelsystem chromatographiert. Die radioaktiven Zonen werden mit einem Detektor lokalisiert. Die dem 6-Keto-$PGF_1\alpha$ (ein stabiles Endprodukt der Prostacyclin-Biotransformation) und $PGE_2$ entsprechenden Zonen werden in Szintillationsröhrchen für Flüssigkeiten übertragen und gezählt. Der Quotient der Zahlenwerte von 6-Keto-$PGF_1\alpha/PGE_2$ wird für jede Konzentration der

verwendeten Testverbindung berechnet. Die $IC_{50}$-Werte der Hemmung werden graphisch ermittelt. Dieser Wert ist die Konzentration der Testverbindung, bei welcher der Quotient von 6-Keto-$PGF_1\alpha$/$PGE_2$ auf 50% des Kontrollwertes reduziert wird.

Die Hemmung der Synthese und die Reduzierung des Plasmaspiegels von Thromboxan wird in vivo dadurch bestimmt, dass man Ratten eine Testverbindung in folgender Weise verabreicht [vgl. auch die von Tai et al., Anal. Biochem. 87, 343 (1978), und von Salmon, Prostaglandins 15, 383 (1978) beschriebenen Verfahren]:

Ratten werden mit einem Trägermaterial bzw. der Testverbindung behandelt; zwei Stunden später wird Ionophor A 23187 (0,5 mg/kg) i.v. injiziert. 2 Minuten nach der Ionophor-Injektion wird das Blut für die Analyse gesammelt. Eine Probe von jedem Plasma wird auf Thromboxan $B_2$ und eine andere Probe auf 6-Keto-$PGF_1\alpha$, den stabilen Metaboliten von Prostacyclin, mittels Radioimmunotest geprüft.

Die Verbindungen der Formel I sind sehr wirksame und selektive Thromboxan-Synthetase-Inhibitoren. Bei wirksamen Dosis-Spiegeln und darüber wird weder das vorteilhafte Prostacyclin-Synthetase- noch das Prostaglandin-cyclooxygenase-Enzymsystem gehemmt. Ueberraschenderweise wird der Prostacyclin-Spiegel signifikant erhöht.

Aufgrund der vorhergenannten vorteilhaften Eigenschaften sind die Verbindungen der Erfindung für Säuger, einschliesslich Menschen, als spezifische therapeutische Mittel, z.B. zur Behandlung kardiovaskulärer Krankheiten, wie Thromboembolie, sehr wertvoll.

Die Verbindungen der vorliegenden Erfindung werden nach an sich bekannten Methoden, vorzugsweise dadurch hergestellt, dass man

A) in Verbindungen der Formel IV

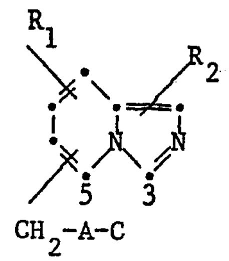

(IV)

oder ihren 5,6,7,8-Tetrahydroderivaten, worin $R_1$, $R_2$ und A die oben angegebene Bedeutung haben und C Carboxy, reaktionsfähiges funktionell abgewandeltes Carboxy, Niederalkoxycarbonyl, Carbamoyl oder Cyan bedeutet, den Rest C in einen Rest B, wie unter der Formel I definiert, überführt.

Nützliche Ausgangsstoffe sind reaktionsfähige funktionelle Derivate von Verbindungen der Formel IV, worin C Carboxy ist, vorzugsweise die Säurehalogenide, einfache oder gemischte Anhydride, z.B. ein Säurechlorid, ein Säureanhydrid $(R_2''CO)_2O$ oder ein gemischtes Anhydrid. Letzteres kann z.B. aus einem Niederalkoxycarbonylhalogenid, z.B. Chlorameisensäureäthylester, oder aus einem sterisch gehinderten Niederalkanoyl-halogenid, z.B. aus Pivaloylchlorid, nach an sich bekannten Methoden hergestellt werden.

Die neuen Zwischenprodukte der Formel IV, worin $R_1$ Hydroxy, Niederalkoxy oder Aryl-niederalkoxy, vorzugsweise Methoxy oder Benzyloxy, ist, $R_2$ Wasserstoff oder Niederalkyl bedeutet, A für Alkylen mit 2 bis 12, vorzugsweise 3 bis 8, Kohlenstoffatomen oder für Alkinylen oder Alkenylen mit 2 bis 12, vorzugsweise mit 4 bis 8 Kohlenstoffatomen steht und C Carboxy, Niederalkoxycarbonyl, Carbamoyl oder Cyan, vorzugsweise Carboxy, bedeutet, und ihre Salze, insbesondere ihre therapeutisch verwendbaren Salze, sind ebenfalls als Thromboxan-Synthetase-Hemmer wirksam.

Verbindungen der Formel I, worin B unsubstiuiertes oder durch Niederalkyl substituiertes 4,5-Dihydro---2-oxazolyl bedeutet, werden vorzugsweise durch die Umsetzung einer Verbindung der Formel IV, worin C für Carboxy oder ein reaktives funktionelles Derivat davon, Niederalkoxycarbonyl oder Carbamoyl steht, mit 2-Hydroxyäthylamin, das gegebenenfalls durch Niederalkyl mono- oder di-(vicinal oder geminal)-substituiert ist, oder mit Aziridin, das gegebenenfalls durch Niederalkyl mono- oder di-(vicinal oder geminal)-substituiert ist, z.B. 2-Aminoäthanol, 2-Methyl-2-aminopropanol oder 2,2-Dimethylaziridin, hergestellt.

Die Kondensation wird nach an sich bekannten Methoden durchgeführt, z.B. wie in J. Org. Chem. 39, 2787 (1974) beschrieben, vorzugsweise in einem inerten Lösungsmittel, z.B. Toluol, in einem Temperaturbereich von 25 bis 100°, und z.B. in der Weise wie in den Beispielen beschrieben.

Die erwähnte Kondensationsreaktion läuft entweder spontan ab, oder es werden, z.B. in dem Fall, wo C für Carboxy steht, Kondensationsmittel, z.B. disubstiuierte Carbodiimide wie etwa Dicyclohexylcarbodiimid, zugesetzt.

Die Verbindungen der Formel I, worin B Hydroxycarbamoyl bedeutet (Hydroxamsäuren), werden vorzugsweise dadurch erhalten, dass man eine Verbindung der Formel IV, worin C Carboxy oder ein reaktionsfähiges funktionelles Derivat davon, Niederalkoxycarbonyl oder Carbamoyl bedeutet, mit Hydroxylamin oder einem Säureadditionssalz davon in Gegenwart einer Base, z.B. Natriumhydroxid, umsetzt.

Diese Reaktion wird in an sich bekannter Weise durchgeführt, z.B. wie von Barton et al., Comprehensive Organic Chemistry, Vol. 2, S. 1037-1038 (1979) beschrieben, vorzugsweise unter basischen Bedingungen, vorteilhaft mit Hydroxylamin-Hydrochlorid, in einem inerten polaren Lösungsmittel, z.B. einem Niederalkanol wie Aethanol,

vorzugsweise in einem Temperaturbereich von 0° bis 50° und vorteilhaft bei Raumtemperatur.

Verbindungen der Formel I, worin B 5-Tetrazolyl bedeutet, werden vorzugsweise dadurch hergestellt, dass man eine Verbindung der Formel IV, worin C vorzugsweise Cyan ist, mit Stickstoffwasserstoffsäure oder einer Verbindung, die Stickstoffwasserstoffsäure in situ bildet, z.B. einem Metall- oder Ammoniumsalz dieser Säure, vorzugsweise einem Alkalimetallazid wie z.B. Natriumazid oder Ammoniumazid, umsetzt.

Diese Kondensation wird in an sich bekannter Weise durchgeführt, z.B. wie von Barton et al., Comprehensive Organic Chemistry, Vol. 4, S. 407-409 (1979) beschrieben, vorzugsweise in einem Lösungsmittel, z.B. Dimethylformamid, und bei erhöhter Temperatur, etwa zwischen 50° und 200°, vorteilhaft zwischen 75° und 150°, und in Gegenwart einer Säure, z.B. Salzsäure oder Ammoniumchlorid.

Die genannten Tetrazolyl-Verbindungen können weiterhin aus einer Verbindung der Formel IV, worin C Cyan oder Carbamoyl ist, hergestellt werden, indem der Rest C zunächst in Halogen- oder Niederalkoxyiminocarbonyl überführt und dann z.B. mit einem Alkalimetall- oder Ammoniumazid umgesetzt wird.

Die Ausgangsstoffe der Formel IV können dadurch hergestellt werden, dass man

1) eine Verbindung der Formel VI

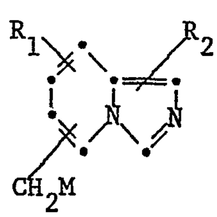

(VI),

worin M ein Alkalimetall bedeutet, $R_1$ Wasserstoff, Niederalkyl,

Niederalkoxy oder Arylniederalkoxy ist und $R_2$ für Wasserstoff oder Niederalkyl steht, mit einem reaktionsfähigen funktionellen Derivat einer Verbindung der Formel VII

HO-A-C' (VII),

worin A Alkylen mit 1 bis 12 Kohlenstoffatomen, Alkinylen oder Alkenylen mit 2 bis 12 Kohlenstoffatomen ist, und C' Carboxy, Trialkoxymethyl, Carbamoyl, Cyan oder Halogenmethyl bedeutet, umsetzt, und eine erhaltene Verbindung der Formel IVa

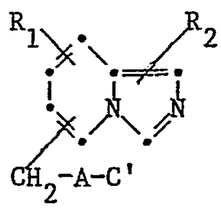

(IVa),

worin A, C', $R_1$ und $R_2$ die oben angegebene Bedeutung haben, und bei der sich C' von C unterscheidet, in eine Verbindung der Formel IV umwandelt.

Reaktionsfähige organometallische Verbindungen der Formel VI, worin M ein Alkalimetallatom bedeutet, können durch Metallierung von geeigneten methyl-substituierten Imidazo[1,5-a]pyridinen erhalten werden. So wird z.B. das gemäss J. Org. Chem. 40, 1210 (1975) hergestellte 5-Methylimidazo[1,5-a]pyridin mit einem reaktionsfähigen Metallierungsmittel, z.B. mit Butyllithium oder Lithium-diisopropylamid, in einem inerten Lösungsmittel, z.B. Tetrahydrofuran, bei einer Temperatur unter Zimmertemperatur, vorzugsweise bei ungefähr -50°, umgesetzt.

Die Kondensation eines Zwischenproduktes der Formel VI mit einem reaktionsfähigen funktionellen Derivat einer Verbindung der Formel VII wird vorzugsweise in einem Temperaturbereich von ungefähr -75° bis +50° vorgenommen. Wenn C' Carboxy bedeutet, wird zunächst ein geeignetes Metallsalz, z.B. das Lithiumsalz, des reaktionsfähigen

funktionellen Derivates der entsprechenden Verbindung der Formel VII hergestellt und dieses mit dem Zwischenprodukt VI umgesetzt.

Ein weiteres Verfahren zur Herstellung von Verbindungen der Formel IV besteht darin, dass man

2) eine Verbindung der Formel VIII

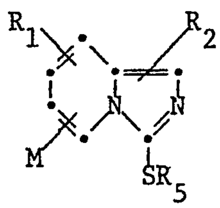

(VIII),

worin M ein Alkalimetall bedeutet, $R_1$ und $R_2$ die oben angegebene Bedeutung haben und $R_5$ Niederalkyl bedeutet, mit einem reaktionsfähigen funktionellen Derivat einer Verbindung der Formel IX

$HOCH_2$-A-C' (IX),

worin A für Alkylen mit 1 bis 12 Kohlenstoffatomen, Alkinylen oder Alkenylen mit 2 bis 12 Kohlenstoffatomen steht, C' Carboxy, Trialkoxymethyl, Carbamoyl, Cyan oder Halogenmethyl bedeutet, umsetzt, in einer erhaltenen Verbindung, worin sich C' von C unterscheidet, die Gruppe C' in C umwandelt und die erhaltene Verbindung desulfuriert.

Die Herstellung des organometallischen Zwischenprodukts VIII und die nachfolgende Kondensation werden wie oben und wie in Tetrahedron Letters 21, 2195-6 (1980) beschrieben, durchgeführt. Die Entschwefelung wird vorzugsweise mit einem Entschwefelungskatalysator wie Raney-Nickel in einem Lösungsmittel, z.B. Aethanol, vorzugsweise bei erhöhter Temperatur vorgenommen.

Weiter können die Verbindungen der Formel IV dadurch hergestellt werden, dass man

3) unter basischer Katalyse eine Verbindung der allgemeinen Formel X

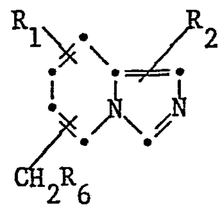

(X),

worin $R_1$ und $R_2$ die oben angegebene Bedeutung haben und $R_6$ für Niederalkoxycarbonyl oder Cyan steht, mit einem reaktionsfähigen funktionellen Derivat einer Verbindung der Formel VII

HO-A-C' (VII),

worin A für Alkylen mit 1 bis 12 Kohlenstoffatomen, Alkinylen oder Alkenylen mit 2 bis 12 Kohlenstoffatomen steht, C' für Carboxy, Trialkoxymethyl, Carbamoyl, Cyan oder Halogenmethyl steht, umsetzt, das Zwischenprodukt hydrolysiert und decarboxyliert, und eine so erhaltene Verbindung, worin sich C' von C unterscheidet, in eine Verbindung der Formel IV umwandelt.

Die Zwischenprodukte der Formel X können durch Behandlung der oben genannten Verbindungen der Formel VI z.B. mit Kohlendioxid und Veresterung der erhaltenen Carbonsäure, oder durch Umsetzung mit einem Di-(niederalkyl)-carbonat oder mit einem Halogencyan, hergestellt werden.

Ein anderes Verfahren zur Herstellung von Verbindungen der Formel IV besteht darin, dass man

4) eine Verbindung der Formel XI

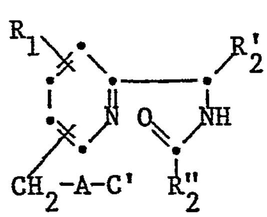

(XI),

worin $R_1$ und A die oben angegebene Bedeutung haben, $R_2'$ und $R_2''$ Wasser-

stoff oder Niederalkyl bedeuten, A die oben angegebene Bedeutung hat, und C' für Carboxy, Niederalkoxycarbonyl, Carbamoyl, Cyan oder Halogenmethyl steht, zu einer Verbindung der Formel IVa ringschliesst und eine erhaltene Verbindung, worin sich C' von C unterscheidet, in eine Verbindung der Formel IV umwandelt.

Der Ringschluss eines Amids der Formel XI wird vorzugsweise unter Bedingungen, wie sie für die Cyclisierung von 6-Methyl-2-methylaminopyridin zu 5-Methylimidazo[1,5-a]pyridin in J. Org. Chem. 40, 1210 (1975) beschrieben sind, durchgeführt. Man verwendet vorteilhaft eine Lewissäure, z.B. Polyphosphorsäure, Phosphoroxychlorid oder Polyphosphat-ester, gegebenenfalls in einem inerten Lösungsmittel, z.B. Toluol, in einem Temperaturbereich zwischen 25° bis 150°, vorzugsweise 50° bis 120°.

Die Amide der Formel XI werden durch Acylierung einer Verbindung der Formel XII

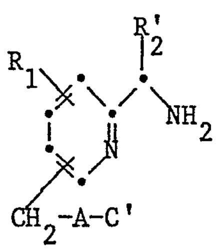

(XII),

worin jedes der Symbole $R_1$, $R_2'$, A und C' die oben angegebene Bedeutung hat, mit einer Carbonsäure der Formel XIII

$R_2''$-COOH (XIII),

worin $R_2''$ die oben angegebene Bedeutung hat, oder mit einem reaktionsfähigen funktionellen Derivat davon, hergestellt.

Reaktionsfähige funktionelle Derivate von Verbindungen XIII sind vorzugsweise Säurehalogenide, einfache oder gemischte Anhydride, z.B. das Säurechlorid, das Säureanhydrid $(R_2''CO)_2O$, oder ein gemischtes

Anhydrid. Letzteres kann z.B. aus einem Niederalkoxycarbonyl-halogenid, z.B. Chlorameisensäureäthylester, oder aus einem gehinderten Niederalkanoyl-halogenid, z.B. aus Pivaloylchlorid, nach an sich bekannten Methoden hergestellt werden.

Die Kondensation von Verbindungen XII und XIII (Acylierung von Verbindungen XII) verläuft entweder spontan, z.B. durch Erhitzen mit Ameisensäure, oder in Gegenwart von Kondensationsmitteln, wie disubstituierten Carbodiimiden, z.B. Dicyclohexylcarbodiimid.

Die Acylierung von Verbindungen XII mit einem reaktionsfähigen funktionellen Derivat von XIII, z.B. mit Acetylchlorid oder Essigsäureanhydrid, wird vorzugsweise in Gegenwart einer organischen oder anorganischen Base, z.B. Kaliumcarbonat oder Triäthylamin, durchgeführt.

Die Amine der Formel XII können z.B. aus entsprechend substituierten 2-(Cyan oder Hydroxyimino-niederalkyl)-pyridinen durch Reduktion, z.B. durch Hydrierung in Gegenwart eines Katalysators wie Palladium auf Kohle, oder durch Behandlung mit einem chemischen Reduktionsmittel, z.B. Boran oder Natriumcyanborhydrid, erhalten werden. Das Reduktionsmittel wird je nach dem Typus der anderen, im Molekül vorhandenen funktionellen Gruppen gewählt. Die Verbindungen der Formel XII können auch durch Aminierung der entsprechend substituierten und reaktionsfähig veresterten 2-(Hydroxymethyl)-pyridine erhalten werden.

Die einzelnen Definitionen in den vorher beschriebenen Verfahren haben die folgenden Bedeutungen:

In einem reaktionsfähigen funktionellen Derivat eines Alkohols der Formel VII und IX ist die Hydroxygruppe z.B. mit einer starken anorganischen Säure oder organischen Sulfonsäure, vor allem mit einer Halogenwasserstoffsäure, z.B. Chlorwasserstoff-, Bromwasserstoff- oder Jodwasserstoffsäure, einer aliphatischen oder aromatischen Sulfonsäure, z.B. Methansulfon- oder p-Toluolsulfonsäure, verestert. Diese Verbindungen werden in an sich bekannter Weise hergestellt.

Trialkoxymethyl bedeutet vorzugsweise Tri(niederalkoxy)-methyl, insbesondere Triäthoxy- oder Trimethoxy-methyl.

Halogenmethyl bedeutet insbesondere Chlormethyl, aber auch Brommethyl oder Jodmethyl.

Ein Alkalimetall ist vorzugsweise Lithium, es kann aber auch Kalium oder Natrium sein.

Die Hydrolyse von Zwischenprodukten, in welchen C' Trialkoxymethyl bedeutet, zu Verbindungen der Formel IV, in welchen C Carboxy ist, wird vorzugsweise mit anorganischen Säuren, wie Halogenwasserstoff- oder Schwefelsäure, vorgenommen.

Zwischenprodukte, z.B. der Formel IVa, in welchen C' Halogenmethyl bedeutet, können vorzugsweise mit einem Metallcyanid, z.B. Kaliumcyanid, in an sich bekannter Weise umgesetzt werden. Man erhält dabei Verbindungen der Formel IV, in welchen die Kette um ein Kohlenstoffatom verlängert ist und C für Cyan steht. Diese können ihrerseits nach an sich bekannten Methoden in Verbindungen der Formel IV, worin C Carboxy oder Alkoxycarbonyl bedeutet, überführt werden.

So können Verbindungen der Formel IV, worin C Cyan bedeutet (Nitrile), in Verbindungen der Formel IV, in welchen C für Carboxy steht, durch Hydrolyse mit anorganischen Säuren, z.B. mit einer Halogenwasserstoffsäure, wie Chlorwasserstoffsäure oder Schwefelsäure, in wässerigen Lösungen oder vorzugsweise durch Hydrolyse mit wässerigen Alkalimetallhydroxiden, z.B. Kaliumhydroxid, vorzugsweise bei Rückflusstemperatur, umgewandelt werden.

Die Umwandlung der genannten Nitrile in Verbindungen der Formel IV, worin C Niederalkoxycarbonyl bedeutet, wird vorzugsweise durch Behandlung mit einem Niederalkanol, z.B. wasserfreiem Aethanol, in Gegenwart

einer starken Säure, z.B. Chlorwasserstoffsäure, vorzugsweise bei Rückflusstemperatur, und nachfolgende vorsichtige Hydrolyse mit Wasser durchgeführt.

Ferner können Zwischenprodukte der Formel IVa, in welchen C' Halogenmethyl, z.B. Chlormethyl, ist, in Verbindungen der Formel IV, worin C Carboxy und die Kettenlänge um zwei Kohlenstoffatome verlängert ist, wie folgt umgewandelt werden: Zuerst wird durch Behandlung mit z.B. einem Malonsäure-di-niederalkylester, wie Malonsäure-diäthylester, in Gegenwart einer Base, z.B. Kaliumcarbonat oder Natriumäthoxid, in einem Lösungsmittel, z.B. Dimethylformamid, vorzugsweise in einem Temperaturbereich zwischen 50 und 100°, ein substituierter Malonsäure-di-niederalkylester hergestellt. Dieser wird vorzugsweise mit einer wässerigenBase, z.B. verdünnter Natronlauge, zu der entsprechenden Malonsäure hydrolysiert und nach Standardbedingungen, z.B. durch Erhitzen in Xylol decarboxyliert, wobei man eine Verbindung der Formel IV, worin C Carboxy bedeutet, erhält. Ersetzt man den Malonsäure-di-niederalkylester durch einen Cyanessigsäure-niederalkylester, so erhält man entsprechende Verbindungen der Formel IV, worin C Cyan bedeutet.

Verbindungen der Formel IV, worin A einen geraden und verzweigten Alkenylenrest mit einer terminalen Doppelbindung bedeutet, können z.B. auch aus Zwischenprodukten der Formel IVa, worin C' Halogenmethyl bedeutet, hergestellt werden. So werden z.B. diese Zwischenprodukte zuerst mit z.B. einem α-(Aryl- oder Alkylthio)-essigsäure. niederalkylester, wie α-(Phenylthio)-essigsäure-äthylester behandelt. Die Reaktion wird in Gegenwart einer starken Base, z.B. Natriumhydrid, durchgeführt. Die nachfolgende Oxidation des erhaltenen α-Arylthio- oder α-Alkylthio-substituierten Esters zum entsprechenden α-Arylsulfinyl- oder α-Alkylsulfinylester mit z.B. Natriumperjodat, und nachfolgende durch Hitze ausgelöste Eliminierung, z.B. durch Kochen in Xylol unter Rückfluss, ergibt eine Verbindung der allgemeinen Formel IV (einen α,β-ungesättigten Ester), worin A Alkenylen

bedeutet und C z.B. für Niederalkoxycarbonyl steht, und die Länge der Kette um 2 Kohlenstoffatome verlängert ist. In ähnlicher Weise können Verbindungen der Formel IVa, worin C' Halogenmethyl bedeutet, zuerst zu den entsprechenden Carboxaldehyden, z.B. mit Dimethylsulfoxid in Gegenwart von Triäthylamin und Silber-tetrafluoroborat, umgewandelt werden. Die nachfolgende Wittig-Kondensation, z.B. mit (Triphenyl-phosphoranyliden)-essigsäureäthylester, ergibt ebenfalls die vorher genannten α,β-ungesättigten Ester.

Verbindungen der Formel IV, in welchen C für unsubstituiertes Carbamoyl steht, können zu den entsprechenden Nitrilen in an sich bekannter Weise, z.B. durch Behandlung mit Phosphoroxychlorid oder Thionylchlorid, in einem inerten Lösungsmittel, z.B. Toluol, dehydratisiert werden.

Freie Carbonsäuren können mit Niederalkanolen, z.B. Aethanol, in Gegenwart einer starken Säure, z.B. Schwefelsäure, vorzugsweise bei erhöhten Temperaturen, oder mit Diazo-niederalkanen, z.B. Diazomethan, in einem Lösungsmittel, z.B. Diäthyläther, vorzugsweise bei Raumtemperatur, zu den entsprechenden Estern, nämlich zu Verbindungen der Formel IV, worin C Niederalkoxycarbonyl bedeutet, verestert werden.

Verbindungen der Formel I können ebenfalls in analoger Weise gemäss den Verfahren 1), 2), 3) und 4), die oben für die Zwischenprodukte der Formel IV beschrieben sind, hergestellt werden. Dabei werden solche Ausgangsstoffe verwendet, die den Formeln VII, IX, XI und XII entsprechen, und worin der Rest C' durch die Gruppe B ersetzt ist, die für 5-Tetrazolyl, Hydroxycarbamoyl oder unsubstituiertes oder durch Niederalkyl substituiertes 4,5-Dihydro-2-oxazolyl steht. Diese Verfahren werden in den Ansprüchen mit B), C), D) und E) bezeichnet. Die Ausgangsstoffe sind bekannt oder können nach an sich bekannten Methoden hergestellt werden.

Verbindungen der Formel I, worin A Alkylen ist, können in die entsprechenden 5,6,7,8-Tetrahydroimidazo[1,5-a]pyridin-Derivate überführt werden, indem sie mit Wasserstoff in Gegenwart eines

Hydrierungskatalysators, z.B. Palladium, und einer Säure, z.B. einer Mineralsäure, wie etwa Salzsäure, in einem inerten Lösungsmittel, z.B. Aethanol, reduziert werden.

Weiter können Verbindungen der Formel I, in welchen A einen geradkettigen oder verzweigten Alkinylen- oder Alkenylenrest bedeutet, durch katalytische Hydrierung, vorzugsweise unter neutralen Bedingungen, z.B. mit einem Palladium-Katalysator bei atmosphärischem Druck in einem inerten Lösungsmittel, z.B. Aethanol, in Verbindungen der Formel I, worin A für geradkettiges oder verzweigtes Alkylen steht, umgewandelt werden.

Verbindungen der Formel I oder IV, worin $R_1$ z.B. Benzyloxy oder Methoxy bedeutet, können in andere Verbindungen der Formel I oder IV, worin $R_1$ Hydroxy ist, z.B. durch Hydrolyse oder Hydrogenolyse nach an sich bekannten Methoden umgewandelt werden.

Ueberdies können Verbindungen der Formel I, worin $R_1$ und $R_2$ Wasserstoff bedeuten, in die entsprechenden Halogenderivate durch direkte Halogenierung mit Chlor, Brom oder Jod, überführt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder anderen oben genannten Mitteln, und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck, und, falls erforderlich, unter vorübergehendem Schutz reaktiver Gruppen, durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder optisch reinen Antipoden verwendet wird.

Im Verfahren der vorliegenden Erfindung werden vorteilhafterweise solche Ausgangsstoffe verwendet, welche zu den im vorstehenden als besonders wertvoll beschriebenen Verbindungen führen.

Die Erfindung betrifft auch die neuen Ausgangsstoffe und Verfahren zu ihrer Herstellung.

Je nach der Wahl von Ausgangsstoffen und Verfahren, können die neuen Verbindungen in Form eines der möglichen Isomeren oder Gemischen von solchen, vorliegen. So können sie z.B. je nach der Anwesenheit einer Doppelbindung und nach der Anzahl der asymmetrischen Kohlenstoffatomen, als reine optische Isomeren, z.B. als Antipoden, oder als Gemische von Isomeren, z.B. als Racemate, Gemische von Diastereomeren, Gemische von Racematen oder Gemische von geometrischen Isomeren sein.

Erhaltene Gemische von Diastereomeren, Gemische von Racematen oder geometrischen Isomeren können auf der Basis der physikochemischen Unterschiede der Komponenten, in an sich bekannter Weise, in die reinen Isomeren, Diasteromeren, Racemate oder geometrischen Isomeren, z.B. durch Chromatographie und/oder fraktionierte Kristallisation, getrennt werden.

Erhaltene Racemate können weiter in die optischen Antipoden in an sich bekannter Weise, z.B. durch Umkristallisation aus optisch aktiven Lösungsmitteln, durch Mikroorganismen, oder durch Umsetzung des saueren Endprodukts mit einer optisch aktiven Base, welche mit der racemischen Säure Salze bildet, aufgetrennt werden. Diese Salze können in dieser Weise, z.B. aufgrund ihrer verschiedenen Löslichkeiten, in die Diastereomeren getrennt werden. Aus den letzteren können die Antipoden durch Einwirkung von geeigneten Mitteln freigesetzt werden. Basische racemische Produkte können in analoger Weise, wie durch Trennung ihrer diastereomeren Salze, z.B. durch fraktionierte Kristallisation der d- oder l-Tartrate, in die Antipoden aufgetrennt werden.

Vorzugsweise wird von den zwei Antipoden der stärker wirksame isoliert.

Schliesslich können die Verbindungen der Erfindung in freier Form oder als Salze erhalten werden. Eine erhaltene freie Base kann in das entsprechende Säureadditionssalz, vorzugsweise mit Säuren, die therapeutisch verwendbare Säureadditionssalze ergeben oder mit Anionenaustauschern, übergeführt werden. Erhaltene Salze können in die entsprechenden freien Basen, z.B. durch Behandlung mit einer stärkeren Base, wie mit einem Metallhydroxid oder Ammoniumhydroxid, basischen Salz, z.B. einem Alkalimetallhydroxid oder -carbonat, oder einem Kationenaustauscher, umgewandelt werden. Eine Verbindung der Formel I, in welcher B 5-Tetrazolyl oder Hydroxycarbamoyl bedeutet, kann auch in die entsprechenden Metall- oder Ammoniumsalze überführt werden. Diese oder andere Salze, z.B. die Pikrate, können auch in der Reinigung von freien Basen verwendet werden. Die Basen werden in ihre Salze übergeführt,die Salze abgetrennt und die Basen aus den Salzen freigesetzt.

Infolge der engen Beziehung zwischen den neuen Verbindungen in freier Form und in Form ihrer Salze sind im vorausgegangenen und nachfolgend unter freien Verbindungen und Salzen sinn- und zweckgemäss gegebenenfalls auch die entsprechenden Salze bzw. freien Verbindungen zu verstehen.

Die Verbindungen und ihre Salze können auch in Form ihrer Hydrate erhalten werden oder andere für die Kristallisation verwendete Lösungsmittel einschliessen.

Die erfindungsgemässen pharmazeutischen Präparate sind solche, die sich für enterale, z.B. orale oder rektale, und parenterale Verabreichung an Säugern, inklusive Menschen, eignen. Die Präparate werden für die Behandlung oder Vorbeugung von Krankheiten, welche auf die Hemmung der Thromboxan-Synthetase, reagieren, z.B. periphäre vaskuläre Krankheiten, verwendet. Diese Präparate enthalten eine wirksame Dosis einer pharmakologisch aktiven Verbindung der Formel I, oder eines pharmazeutisch verwendbaren Salzes davon, allein oder in Kombination mit einem oder mehreren pharmazeutisch annehmbaren Trägermaterialien.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, oder Brausemischungen, und/oder Absorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmakolgischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeütischen Präparate, die, wenn erwünscht, weitere pharmakologisch wertvolle

Stoffe enthalten können, werden in an sich bekannter Weise, z.B. mittels konventioneller Misch-, Granulier- oder Dragierverfahren, hergestellt und enthalten von etwa 0,1% bis etwa 75%, insbesondere von etwa 1% bis etwa 50%, des Aktivstoffes. Einzeldosen für Säuger mit einem Gewicht von ungefähr 50-70 kg können zwischen ungefähr 20-200 mg des aktiven Bestandteils enthalten.

Die folgenden Beispiele dienen zur Illustration der Erfindung und sie sind nicht als Einschränkung ihres Umfangs aufzufassen. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z.B. zwischen ungefähr 20 und 130 mbar, durchgeführt. In den Beispielen 1 - 28 wird die Herstellung einiger repräsentativer Ausgangsstoffe, z.B. der Formel IV und IVa, beschrieben.

Beispiel 1: Eine Lösung von 50 g 5-Methylimidazo[1,5-a]pyridin [J. Org. Chem. 40, 1210 (1975)] in 625 ml Tetrahydrofuran wird auf -75° vorgekühlt und unter Stickstoff mit 175 ml 2,4-normalem Butyllithium in Hexan versetzt, wobei man die Temperatur unter -53° hält. Die Lösung von 5-(Lithiomethyl)-imidazo[1,5-a]pyridin wird wieder auf -75° gekühlt und mit einer Lösung von 121,8 g 5-Brom-1,1,1-triäthoxypentan in 125 ml Tetrahydrofuran rasch versetzt, wobei die Temperatur auf -60° steigt. Man lässt das Reaktionsgemisch innerhalb 45 Minuten sich auf -4° erwärmen und dampft es praktisch zur Trockene ein. Der Rückstand wird zwischen 500 ml Aethyläther und 240 ml 3-normaler Chlorwasserstoffsäure verteilt. Die Aetherlösung wird weiter zweimal mit 60 ml 3-normaler Chlorwasserstoffsäure extrahiert. Die vereinigten wässerigen Extrakte werden mit 100 ml konz. Ammoniumhydroxid basisch gemacht und zweimal mit 200 ml Aethyläther extrahiert. Der Aetherextrakt wird über Magnesiumsulfat getrocknet und zur Trockene eingedampft. Das erhaltene Oel wird im Hochvakuum destilliert. Man erhält das 5-(5-Aethoxycarbonylpentyl)-imidazo[1,5-a]pyridin, welches bei 180-186°/0,16 mbar siedet.

Beispiel 2: Eine Suspension von 26 g 5-(5-Aethoxycarbonylpentyl)-imidazo[1,5-a]pyridin in 100 ml 1-normaler wässeriger Natriumhydroxidlösung wird zwei Stunden auf dem Dampfbad erhitzt, mit 10 ml Aethanol versetzt und 45 Minuten weiter erhitzt. Das Reaktionsgemisch wird abgekühlt, mit 300 ml Aether gewaschen und die Lösung mit konz. Chlorwasserstoffsäure auf den pH-Wert 5,5 eingestellt. Das kristallisierte Produkt wird abfiltriert und mit 50 ml Wasser gewaschen. Man erhält das 5-(5-Carboxypentyl)-imidazo[1,5-a]pyridin, welches bei 144-147° schmilzt.

Beispiel 3: a) Eine Lösung von 39,6 g 5-Bromvaleriansäure in 400 ml Tetrahydrofuran wird auf -78° gekühlt und langsam mit 93 ml 2,3-normaler Butyllithium-Lösung in Hexan versetzt, wobei man die Temperatur unter -65° hält. Die Suspension wird 20 Minuten gerührt und dann mit einer Lösung von 5-(Lithiomethyl)-imidazo[1,5-a]pyridin (hergestellt aus 26,9 g 5-Methylimidazo[1,5-a]pyridin und 93 ml 2,3-normaler n-Butyllithium-Lösung gemäss Beispiel 1) auf einmal bei -75° versetzt. Das Reaktionsgemisch wird bei -75° zwei Stunden gerührt, sich auf Zimmertemperatur erwärmen gelassen, mit 15 ml 12-normaler Chlorwasserstoffsäure behandelt und im Vakuum eingedampft.

Der Rückstand wird zwischen Wasser und Methylenchlorid verteilt, nachdem man den pH-Wert mit Natriumcarbonat auf 10 eingestellt hat. Die wässerige Lösung wird mit Chloroform weiter gewaschen, mit 12-normaler Chlorwasserstoffsäure auf den pH-Wert 1 eingestellt und wieder mit Aether und Toluol gewaschen. Der pH-Wert wird mit Natriumhydrogencarbonat auf 5,5 eingestellt, und die Extraktion mit Chloroform ergibt das rohe 5-(5-Carboxypentyl)-imidazo[1,5-a]pyridin. Eine Lösung der Säure in 30 ml Acetonitril wird mit 20 ml 5-normaler Chlorwasserstoffsäure behandelt. Nach Zugabe von 25 ml Aethyläther erhält man das kristalline 5-(5-Carboxypentyl)-imidazo[1,5-a]pyridin-hydrochlorid, welches bei 201-204° schmilzt. Das 5-(5-Carboxypentyl)-imidazo[1,5-a]-pyridin (Beispiel 2) wird nach dem Neutralisieren der methanolischen Lösung mit verdünnter Natriumhydroxidlösung auf den pH-Wert 5 erhalten.

b) Ausgehend von 6-Bromhexansäure wird in analoger Weise das 5-(6-Carboxyhexyl)-imidazo[1,5-a]pyridin, welches bei 137-139° schmilzt, hergestellt.

c) In analoger Weise wird auch das 5-(7-Carboxyheptyl)-imidazo[1,5-a]-pyridin ausgehend von 7-Bromheptansäure erhalten. F. 97-101°.

Beispiel 4: Eine Lösung von 37 g 5-(5-Chlorpentyl)-imidazo[1,5-a]-pyridin, 21,7 g Kaliumcyanid und 3 g Dibenzo-18-kronenäther-6 in 500 ml Acetonitril wird 20 Stunden unter Rückfluss gekocht. Das Acetonitril wird unter vermindertem Druck abgedampft, der Rückstand zwischen Wasser und Methylenchlorid verteilt und der Methylenchlorid-Extrakt zur Trockene eingedampft. Durch Behandlung einer Lösung des Rückstands in Aether mit äthanolischer Chlorwasserstoffsäure erhält man das 5-(5-Cyanpentyl)-imidazo[1,5-a]pyridin-hydrochlorid, welches bei 178-180° schmilzt.

Der Ausgangsstoff wird wie folgt hergestellt:
Eine Lösung von 30 g 1-Brom-4-chlorbutan in 20 ml trockenem Tetrahydrofuran wird zu einer Lösung von 5-(Lithiomethyl)-imidazo[1,5-a]-pyridin (hergestellt gemäss Beispiel 1 aus 22 g 5-Methyl-imidazo[1,5-a]-pyridin und 80 ml einer 2,3-normalen Lösung von n-Butyllithium in Hexan) gegeben, wobei man die Temperatur unter -50° hält. Das Reaktionsgemisch wird 2-3 Stunden bei -50° gerührt, auf Zimmertemperatur erwärmt, über Nacht gerührt und zur Trockene eingedampft. Die Lösung des Rückstandes in 200 ml Methylenchlorid wird mit Wasser gewaschen, über Magnesiumsulfat getrocknet und zur Trockene eingedampft.
Man erhält das 5-(5-Chlorpentyl)-imidazo[1,5-a]pyridin, welches ohne weitere Reinigung verwendet wird.

Beispiel 5: Gemäss dem im Beispiel 4 beschriebenen Verfahren wird das 5-(4-Chlorbutyl)-imidazo[1,5-a]pyridin in das 5-(4-Cyanbutyl)-imidazo[1,5-a]pyridin umgewandelt. F. 72-77°.

Beispiel 6: In einem dem im Beispiel 4 beschriebenen analogen Verfahren wird das 3,5-Dimethylimidazo[1,5-a]pyridin (J. Het. Chem. 3, 33 (1966)] in das 5-(5-Chlorpentyl)-3-methyl-imidazo[1,5-a]pyridin umgewandelt. F. 98-104°. Die gemäss den Bedingungen des Beispiels 4 durchgeführte Umsetzung mit Kaliumcyanid ergibt das 5-(5-Cyanpentyl)-3-methyl-imidazo[1,5-a]pyridin, welches in sein Hydrobromid durch Auflösen der freien Base in Acetonitril und Ansäuern der Lösung mit äthanolischem Bromwasserstoff übergeführt wird. Das erhaltene 5-(5-Cyanpentyl)-3-methylimidazo[1,5-a]pyridin-hydrobromid schmilzt bei 215-220°.

Beispiel 7: Eine Lösung von 36 g 5-(Cyanpentyl)-imidazo[1,5-a]-pyridin in 100 ml Methanol und 50 ml einer 45%-igen wässerigen Kaliumhydroxidlösung wird unter Rückfluss 48 Stunden gekocht. Das Methanol wird unter vermindertem Druck abgedampft und der Rückstand mit Wasser versetzt. Die basische Lösung wird mit Essigsäureäthylester gewaschen und mit konz. Chlorwasserstoffsäure auf einen pH-Wert von 5,5-6 angesäuert.

Die kristallisierte Säure wird abgetrennt und aus Aethanol umkristallisiert. Man erhält das Produkt des Beispiels 2, nämlich das 5-(5-Carboxypentyl)-imidazo[1,5-a]pyridin, welches bei 142-145° schmilzt. Eine weitere Umkristallisation erhöht den Schmelzpunkt auf 144-147°.

Beispiel 8: Die Hydrolyse des 5-(4-Cyanbutyl)-imidazo[1,5-a]pyridins gemäss Beispiel 7 ergibt das 5-(4-Carboxybutyl)-imidazo[1,5-a]pyridin, welches bei 161-163° schmilzt.

Beispiel 9: Die Hydrolyse des 5-(5-Cyanpentyl)-3-methyl-imidazo[1,5-a]-pyridins gemäss Beispiel 7 ergibt das 5-(5-Carboxypentyl)-3-methyl-imidazo[1,5-a]pyridin, welches bei 170-173° schmilzt.

Beispiel 10: Eine Lösung von 3 g 5-(5-Cyanpentyl)-3-methyl-imidazo-[1,5-a]pyridin-hydrochlorid in einem Gemisch von 20 ml Aethanol und 5 ml 1-normaler wässeriger Natriumhydroxidlösung wird mit 10 ml einer 30%-igen Wasserstoffsuperoxidlösung versetzt. Das Reaktionsgemisch wird dann mit 5 ml Aethanol versetzt und mit 1-normaler Natriumhydroxidlösung auf den pH-Wert 10 eingestellt.

Das Gemisch wird über Nacht bei Zimmertemperatur gerührt, das Aethanol unter vermindertem Druck abgedampft, der Rückstand mit Wasser versetzt und mit Methylenchlorid extrahiert. Das erhaltene Produkt wird aus Aether kristallisiert und aus Acetonitril umkristallisiert. Man erhält das 5-(5-Carbamoylpentyl)-imidazo[1,5-a]pyridin, welches bei 131-132° schmilzt.

Beispiel 11: a) Eine Lösung von 2,7 g 5-(6-Carboxyhexyl)-imidazo-[1,5-a]pyridin in einem Gemisch von 120 ml Aethanol und 30 ml konz. Chlorwasserstoffsäure wird bei 3 Atmosphären in Gegenwart von 1 g 10%-igem Palladium-auf-Kohle Katalysator bis zur Aufnahme von 2 Mol Wasserstoff hydriert. Das Gemisch wird vom Katalysator abfiltriert und zur Trockene eingedampft. Der Rückstand wird aus Isopropanol/Aether umkristallisiert. Man erhält das 5-(6-Carboxyhexyl)-5,6,7,8-tetrahydroimidazo[1,5-a]pyridin-hydrochlorid, welches bei 150-154° schmilzt.

b) In analoger Weise erhält man durch Hydrierung von 5-(5-Carboxypentyl)-imidazo[1,5-a]pyridin das 5-(5-Carboxypentyl)-5,6,7,8-tetrahydroimidazo[1,5-a]pyridin-hydrochlorid, welches bei 146-150° schmilzt.

c) Ausgehend von 5-(4-Carboxybutyl]-imidazo[1,5-a]pyridin erhält man in analoger Weise das 5-(4-Carboxybutyl)-5,6,7,8-tetrahydro-imidazo-[1,5-a]pyridin-hydrochlorid, welches bei 120-123° schmilzt.

Beispiel 12: Eine Lösung von 2,3 g (0,011 Mol) 5-Brom-3,3-dimethylpentansäure [J. Org. Chem. 44, 1258 (1979)] in 20 ml trockenem Tetrahydrofuran wird in einer Stickstoffatmosphäre auf -70° gekühlt und mit 5,05 ml 2,4-molarem n-Butyllithium in Hexan tropfenweise versetzt. Nach dem Abschluss der Zugabe wird eine Lösung von 5-(Lithiomethyl)-imidazo[1,5-a]pyridin in Hexan (hergestellt aus 1,32 g 5-Methylimidazo[1,5-a]pyridin und 5,05 ml 2,4-normalem n-Butyllithium in Hexan) auf einmal zugegeben. Das Gemisch wird über Nacht bei Zimmertemperatur gerührt.

Das Reaktionsgemisch wird mit 50 ml Wasser verdünnt, mit 10 g Natriumcarbonat versetzt und die basische Lösung wird dreimal mit je 75 ml Chloroform extrahiert. Die wässerige Phase wird mit 12-normaler Chlorwasserstoffsäure auf den pH-Wert 2 angesäuert und dreimal mit je 100 ml Aether extrahiert. Schliesslich wird die wässerige Phase mit verdünnter Natriumhydroxidlösung auf den pH-Wert 5 eingestellt und mit 200 ml eines Essigsäureäthylester/Aether-Gemisches (1:1) extrahiert. Die Extrakte werden getrocknet und eingedampft, wobei man ein gelbes Oel erhält. Dieses wird aus 50 ml eines Aethanol/Aether-Gemisches (1:1) umkristallisiert. Man erhält das 5-(5-Carboxy-4,4-dimethylpentyl)-imidazo[1,5-a]pyridin, welches bei 124-129° schmilzt.

Beispiel 13: Man gibt 1,9 g Jodkristalle zu einer stark gerührten Lösung von 1,16 g 5-(5-Carboxypentyl)-imidazo[1,5-a]pyridin und 1,68 g Natriumbicarbonat in 10 ml Wasser und 1 ml Aethanol. Zur Auflösung des Grossteils von Jod gibt man weitere 4 ml Aethanol dazu und rührt das Gemisch weitere 45 Minuten. Das Reaktionsgemisch wird mit 12 ml Wasser verdünnt und zweimal beim pH-Wert 8 (falls nötig unter Zugabe von Natriumhydrogencarbonat) mit Methylenchlorid extrahiert. Die wässerige Phase wird im Vakuum konzentriert, mit Aktivkohle gereinigt und mit 2-normaler Chlorwasserstoffsäure auf den pH-Wert 4,5 eingestellt. Der Niederschlag wird abgetrennt, getrocknet und aus Methanol/Aether umkristallisiert. Man erhält das 1-Jod-5-(5-carboxypentyl)-imidazo[1,5-a]pyridin, welches bei 163-165° schmilzt.

Beispiel 14: Eine Lösung von 5-Methylimidazo[1,5-a]pyridin (4,0 g) und Tetramethyl-äthylendiamin (4,9 g) in 100 ml Tetrahydrofuran wird unter Stickstoff auf 0° gekühlt und mit 26,5 ml 1,6-normaler n-Butyllithium in Hexan tropfenweise versetzt, wobei man die Temperatur unter 2° hält. Diese Lösung wird nach 30 Minuten, in einer Stickstoffatmosphäre, innerhalb 45 Minuten zu einer eiskalten Lösung von 5-Brom-valeronitril (4,86 g) in 80 ml Tetrahydrofuran gegeben. Das Lösungsmittel wird nach 15 Minuten abgedampft und der Rückstand zwischen Wasser und Essigsäureäthylester verteilt. Die organische Phase wird mit 2-normaler Chlorwasserstoffsäure (3 x 15 ml) extrahiert. Die wässerige Phase wird mit 50%-iger Natriumhydroxidlösung auf den pH-Wert 10 eingestellt. Man extrahiert mit Essigsäureäthylester (2 x 75 ml), trocknet mit Magnesiumsulfat, dampft ein und chromatographiert (Silicagel, Essigsäureäthylester). Man erhält das 5-(5-Cyanpentyl)-imidazo[1,5-a]pyridin.

Beispiel 15: Eine Lösung von 4 g 5-(4-Aethoxycarbonylbutyl)-3-äthylthio-imidazo[1,5-a]pyridin in 100 ml Aethanol wird mit ungefähr 5 g Raney-Nickel versetzt. Die Lösung wird 18 Stunden unter Rückfluss gekocht. Das Raney-Nickel wird abfiltriert und mit 100 ml Essigsäureäthylester gewaschen. Das Filtrat wird unter vermindertem Druck zur Trockene eingedampft. Man erhält das Produkt als ein schweres Oel. Dieses wird durch Säulenchromatographie (Silicagel) gereinigt und mit einem (1:3)-Gemisch von Aether-Hexan eluiert. Das Lösungsmittel wird unter vermindertem Druck eingedampft. Man erhält das 5-(4-äthoxycarbonyl-butyl)-imidazo[1,5-a]pyridin als gelbes Oel. NMR ($CDCl_3$) 1,25 (t, 3H), 4,15 (q, 2H), 8,1 (s, 1H).

Der Ausgangsstoff wird wie folgt hergestellt:
Man löst 17,8 g 3-Aethylthio-imidazo[1,5-a]pyridin in 200 ml trockenem Tetrahydrofuran und kühlt die Lösung auf -70°. Innerhalb 15 Minuten gibt man tropfenweise, unter Rühren, 80 ml von 1,6-molarem n-Butyllithium in Hexan dazu. Nach beendeter Zugabe wird das Reaktionsgemisch weitere 30 Minuten bei -70° gerührt. Das Gemisch wird mit einer Lösung von 20 g 4-Brompentansäure-äthylester in 75 ml Tetrahydrofuran tropfenweise versetzt. Das Reaktionsgemisch lässt man sich auf -10° erwärmen, hält es 30 Minuten bei dieser Temperatur und nachfolgend 1 Stunde bei Raumtemperatur. Das Reaktionsgemisch wird mit 400 ml Diäthyläther und 400 ml 4-normaler Chlorwasserstoffsäure versetzt. Die wässerige Phase wird abgetrennt und die ätherische Schicht mit Wasser gewaschen. Die vereinigten wässerigen Extrakte werden mit Ammoniumhydroxid basisch gemacht und dreimal mit je 200 ml Aether extrahiert. Der Aetherextrakt wird über wasserfreiem Magnesiumsulfat getrocknet und das Lösungsmittel bei vermindertem Druck abgedampft. Als Rohprodukt erhält man ein schweres Oel. Dieses wird auf einer Silicagelsäule chromatographiert und mit einem (4:1)-Gemisch von Pentan-Diäthyläther eluiert. Das Lösungsmittel wird abgedampft und das Produkt destilliert. Man erhält das 3-Aethylthio-5-(4-äthoxycarbonylbutyl)-imidazo[1,5-a]-pyridin, welches bei 170°/0,4 mbar siedet. NMR ($CDCl_3$) 1,25 (t, 3H), 1,30 (t, 3H), 3,15 (q, 2H), 4,15 (q, 2H).

Beispiel 16: Eine Lösung von 3 g 5-[5-Aethoxycarbonyl-5-(phenylsulfinyl)-pentyl]-imidazo[1,5-a]pyridin in 50 ml Xylol wird unter Stickstoff 30 Minuten unter Rückfluss gekocht. Das Xylol wird dann unter vermindertem Druck abgedampft, der Rückstand in 15 ml Diäthyläther gelöst und auf Silicagel chromatographiert. Das Produkt wird mit einem (2:1)-Gemisch von Diäthyläther und Essigsäureäthylester eluiert. Nach Abdampfen des Lösungsmittels erhält man das 5-(5-Aethoxycarbonylpent-4-enyl)-imidazo[1,5-a]pyridin als ein Oel. NMR ($CDCl_3$) 1,29 (t, 3H), 4,25 (q, 2H), 5,88 (d, 1H).

Der Ausgangsstoff wird wie folgt hergestellt:
Eine eisgekühlte, magnetisch gerührte Aufschwemmung von 0,96 g Natriumhydrid in 50 ml Dimethylformamid wird innerhalb 15 Minuten mit 3,92 g 2-(Phenylthio)-essigsäureäthylester tropfenweise versetzt. Die Suspension wird bei Zimmertemperatur 2 Stunden gerührt und dann mit

einem Eisbad auf 5° gekühlt. Diese Suspension wird innerhalb 1 Stunde mit 4,16 g 5-(4-Chlorbutyl)-imidazo[1,5-a]pyridin tropfenweise versetzt. Dann wird das Gemisch mit 3,2 g Natriumjodid versetzt und über Nacht bei Zimmertemperatur gerührt.

Das Reaktionsgemisch wird in 150 ml Eiswasser gegossen und dreimal mit je 100 ml eines (1:1)-Gemisches von Diäthyläther und Essigsäureäthylester extrahiert. Die organische Phase wird zweimal mit je 100 ml gesättigter wässeriger Natriumchloridlösung gewaschen und dann dreimal mit je 50 ml 1-normaler Chlorwasserstoffsäure extrahiert. Die saueren wässerigen Extrakte werden vereinigt, mit Ammoniumhydroxid basisch gemacht und dreimal mit je 150 ml eines 1:1-Gemisches von Diäthyläther und Essigsäureäthylester extrahiert. Die organischen Extrakte werden über wasserfreiem Magnesiumsulfat getrocknet, filtriert und unter vermindertem Druck eingedampft. Man erhält ein Oel, welches durch Säulenchromatographie auf Silicagel gereinigt und mit Diäthyläther eluiert wird. Nach Abdampfen des Lösungsmittels erhält man das 5-[5-Aethoxycarbonyl-5-(phenylthio)-pentyl]-imidazo[1,5-a]-pyridin als ein schweres Oel. NMR ($CDCl_3$) 3,3-3,8 (1H); IR 1720 $cm^{-1}$.

Eine Lösung von 3,8 g 5-[5-Aethoxycarbonyl-5-(phenylthio)-pentyl]-imdiazo[1,5-a]pyridin in 100 ml Methanol wird mit 2,8 g Natrium-metaperjodat versetzt und das Gemisch 18 Stunden bei Raumtemperatur gerührt. Das Lösungsmittel wird unter vermindertem Druck abgedampft und der Rückstand in 150 ml Wasser aufgenommen. Man extrahiert dreimal mit je 100 ml Essigsäureäthylester. Die organische Phase wird zweimal mit je 50 ml 1-normaler Chlorwasserstoffsäure extrahiert, der wässerige Extrakt mit Ammoniumhydroxid basisch gemacht und wieder mit 2 x 100 ml Essigsäureäthylester extrahiert. Die vereinigten Essigsäureäthylester-Extrakte werden über wasserfreiem Magnesiumsulfat getrocknet, filtriert und unter vermindertem Druck eingedampft. Man erhält ein Oel, welches durch Säulenchromatographie auf Silicagel gereinigt und mit einem (1:1)-Gemisch von Essigsäureäthylester

und Diäthyläther eluiert wird. Nach Eindampfen erhält man das 5-[5-Aethoxycarbonyl-5-(phenylsulfinyl)-pentyl]-imidazo[1,5-a]pyridin als ein Oel. IR 1720 $cm^{-1}$, 1040 $cm^{-1}$.

Beispiel 17: Eine Lösung von 300 mg 5-(5-Aethoxycarbonylpent-4-enyl)-imidazo[1,5-a]pyridin in 20 ml Methanol wird mit 5 ml 1-normaler Natriumhydroxidlösung versetzt und das Gemisch 18 Stunden bei Raumtemperatur gerührt. Das Methanol wird unter vermindertem Druck abgedampft und der wässerige Rückstand mit weiteren 5 ml Wasser versetzt. Das Gemisch wird dreimal mit je 5 ml Essigsäureäthylester extrahiert. Die basische wässerige Schicht wird dann auf den pH-Wert 5 eingestellt und dreimal mit je 5 ml Essigsäureäthylester extrahiert. Diese Extrakte werden über wasserfreiem Natriumsulfat getrocknet, filtriert und unter vermindertem Druck eingedampft. Man erhält das 5-(5-Carboxypent-4-enyl)-imidazo[1,5-a]pyridin, welches bei 142-144° schmilzt.

Beispiel 18: Zu einer Lösung von 2,75 g 5-(5-Formylpentyl)-imidazo-[1,5-a]pyridin in 180 ml Chloroform gibt man 6,5 g Carbäthoxymethylentriphenylphosphoran und rührt das Gemisch 18 Stunden bei Raumtemperatur. Das Lösungsmittel wird dann unter vermindertem Druck abgedampft. Man erhält das 5-(7-Aethoxycarbonyl-hept-6-enyl)-imidazo[1,5-a]pyridin als ein Oel.

Der Ausgangsstoff wird wie folgt hergestellt:
Eine auf -60° gekühlte Lösung von 4,9 g 5-(5-Methoxycarbonyl-pentyl)-imidazo[1,5-a]pyridin (welches durch Veresterung des im Beispiel 2 hergestellten 5-(5-Carboxypentyl)-imidazo[1,5-a]pyridins mit Diazomethan in Methylenchlorid erhalten wird) in 140 ml Methylenchlorid wird innerhalb 20 Minuten mit 40 ml einer 1,75-molaren Lösung von Di-isobutyl-aluminiumhydrid in Hexan tropfenweise versetzt. Dann wird das Reaktionsgemisch 20 Minuten bei -60° gerührt und nachfolgend mit 10 ml Methanol und 100 ml Wasser zur Unterbrechung der Reaktion versetzt. Das Gemisch wird bei Raumtemperatur 15 Minuten gerührt, die

Methylenchlorid-Schicht abgetrennt und das Lösungsmittel bei vermindertem Druck abgedampft. Man erhält das 5-(5-Formylpentyl)-imidazo-[1,5-a]pyridin als ein Oel. NMR ($CDCl_3$) 9,7 (m, 1H); IR (Methylenchlorid) 1710 $cm^{-1}$.

Beispiel 19: Eine Lösung von 2,8 g 5-(7-Aethoxycarbonyl-hept-6-enyl)-imidazo[1,5-a]pyridin in 30 ml Methanol wird mit 15 ml 1-normaler Natriumhydroxidlösung versetzt und das Gemisch bei Raumtemperatur 3 Stunden gerührt. Das Methanol wird unter vermindertem Druck abgedampft, der Rückstand mit 30 ml Wasser verdünnt und die Lösung mit 1-normaler Chlorwasserstoffsäure auf den pH-Wert 7 eingestellt. Die Lösung wird mit 2 x 50 ml Essigsäureäthylester extrahiert. Die vereinigten Extrakte werden über wasserfreiem Magnesiumsulfat getrocknet, filtriert und das Lösungsmittel unter vermindertem Druck abgedampft. Man erhält das 5-(7-Carboxyhept-6-enyl)-imidazo[1,5-a]pyridin, welches bei 110-111° schmilzt.

Beispiel 20: Eine Lösung von 0,1 g 2-Aminomethyl-3-(4-methoxycarbonylbutyl)-pyridin in 0,6 ml Ameisensäure wird 18 Stunden bei 90° erhitzt. Das Gemisch wird auf 0° gekühlt, mit gesättigter wässeriger Ammoniumhydroxidlösung basisch gemacht und mit Methylenchlorid (4 x 10 ml) extrahiert. Die Extrakte werden getrocknet, filtriert und eingedampft. Man erhält das 2-(N-Formylaminomethyl)-3-(4-methoxycarbonylbutyl)-pyridin, welches bei 43-45° schmilzt. Dieses wird in 1 ml Toluol wieder aufgelöst und 17 Stunden bei 90° mit 75 mg Phosphoroxychlorid erhitzt. Das überschüssige Phosphoroxychlorid wird mit Toluol abgedampft, der Rückstand bei 0° mit gesättigter wässeriger Ammoniumhydroxidlösung basisch gemacht, mit Methylenchlorid (4 x 15 ml) extrahiert und der Extrakt über Natriumsulfat getrocknet. Nach Eindampfen erhält man ein Oel, dass nach Chromatographie (Silicagel, Essigsäureäthylester) ein Oel ergibt. Dieses ist das 8-(4-Methoxycarbonylbutyl)-imidazo[1,5-a]-pyridin. Rf = 0,29; NMR ($CDCl_3$) 3,70 (s, 3H), 6,50 (d, 2H), 7,43 (s, 1H), 7,83 (t, 1H), 8,22 (s, 1H); IR (Methylenchlorid) 1725 $cm^{-1}$.

Der Ausgangsstoff wird wie folgt hergestellt:
Eine Lösung von 3-Brompyridin (7,9 g), 4-Pentensäure-methylester (7,15 g), Palladiumacetat (0,11 g) und Tri-o-tolylphosphin (0,6 g) in 50 ml Triäthylamin wird 24 Stunden unter Argon unter Rückfluss gekocht und das Lösungsmittel abgedampft. Der Rückstand wird in Methylenchlorid aufgenommen (50 ml) und mit Wasser (2 x 40 ml) gewaschen. Die organische Phase wird getrocknet und eingedampft. Man erhält das 3-(4-Methoxycarbonylbut-1-enyl)-pyridin als eine farblose Flüssigkeit. NMR ($CDCl_3$) 3,72 (s, 3H), 6,40 (s, 1H); IR (Film) 1725 $cm^{-1}$.

3-(4-Methoxycarbonylbut-1-enyl)-pyridin (9,5 g) wird in 100 ml Methanol mit 0,5 g 5%-igem Palladium-auf-Kohle-Katalysator 3,5 Stunden bei 3 Atmosphären hydriert. Nach Filtrierung und Eindampfen erhält man das 3-(4-Methoxycarbonylbutyl)-pyridin als ein Oel. NMR ($CDCl_3$) 3,80 (s, 3H); IR (Methylenchlorid) 1730 $cm^{-1}$.

3-(4-Methoxycarbonylbutyl)-pyridin (10,81 g) wird tropfenweise, wobei man die Temperatur des Reaktionsgemisches zwischen 80-85° hält, mit Peressigsäure (40%, 8,3 ml) versetzt. Nachher lässt man die Temperatur auf 30° sinken und die überschüssige Peressigsäure wird mit wässeriger Natriumsulfitlösung zerstört. Die Essigsäure wird unter vermindertem Druck abgedampft, der Rückstand in Methylenchlorid (50 ml) aufgenommen, filtriert und eingedampft. Der Rückstand, der aus 3-(4-Methoxycarbonylbutyl)-pyridin-N-oxid besteht, wird mit Dimethylsulfat (7,7 g) in 40 ml Toluol bei 90° eine Stunde behandelt und das Lösungsmittel abgedampft. Das 3-(4-Methoxycarbonylbutyl)-1-methoxypyridinium-methylsulfat-Salz wird in 16,7 ml eiskaltem Wasser und 8,3 ml 1-normaler Natriumhydroxidlösung gelöst und mit einer Lösung von Kaliumcyanid (11,21 g) in 16,7 ml eiskaltem Wasser langsam, wobei man die Temperatur bei 0° hält, versetzt. Nach 24 Stunden bei 0°, extrahiert man mit Methylenchlorid (3 x 30 ml), trocknet den Extrakt über Natriumsulfat und dampft das Lösungsmittel ab. Man erhält ein Gemisch von isomeren Cyanpyridinen, von welchen man das

2-Cyan-3-(4-methoxycarbonylbutyl)-pyridin mit dem Rf-Wert von 0,56 und NMR ($CDCl_3$) 8,82 (m, 1H) und das 2-Cyan-5-(4-methoxycarbonylbutyl)-pyridin mit dem Rf-Wert 0,50 und NMR ($CDCl_3$) 8,72 (s, 1H) durch Chromatographie getrennt werden (Silicagel, Aether-Pentan 3:2).

2-Cyan-3-(4-methoxycarbonylbutyl)-pyridin (2,40 g) wird in 92 ml Methanol, welches 2,4 ml konz. Chlorwasserstoffsäure enthält, gelöst und mit 1,2 g 10%-igem Palladium-auf-Kohle-Katalysator 3 Stunden bei atmosphärischem Druck hydriert. Filtrieren, Eindampfen und Umkristallisation aus Aether-Methylenchlorid ergibt das 2-Aminomethyl-3-(4-methoxycarbonylbutyl)-pyridin-hydrochlorid, welches bei 79-81° schmilzt.

Beispiel 21: Eine Lösung von 8-(4-Methoxycarbonylbutyl)-imidazo[1,5-a]-pyridin (30 mg) in 0,3 ml Aethanol und 0,3 ml 1-normaler Natriumhydroxidlösung wird 2 Stunden unter Rückfluss gekocht, abgekühlt, mit 2 ml Wasser verdünnt und mit Essigsäureäthylester (1 x 5 ml) extrahiert. Die wässerige Phase stellt man auf den pH-Wert 6 ein und extrahiert mit Methylenchlorid (4 x 10 ml). Die Extrakte werden getrocknet und eingedampft. Man erhält das 8-(4-Carboxybutyl)-imidazo-[1,5-a]pyridin, welches bei 195-197° schmilzt.

Beispiel 22: 2-Aminomethyl-5-(4-methoxycarbonylbutyl)-pyridin (0,20 g) wird in 0,6 ml Ameisensäure 18 Stunden auf 90° erhitzt. Das Gemisch wird auf 0° gekühlt, mit gesättigter wässeriger Ammoniumhydroxidlösung basisch gemacht und mit Methylenchlorid (4 x 15 ml) extrahiert. Die Extrakte werden getrocknet, filtriert und eingedampft. Man erhält das 2-(N-Formylaminomethyl)-5-(4-methoxycarbonylbutyl)-pyridin als ein Oel (IR 1720, und 1675 $cm^{-1}$), welches in 1 ml Toluol gelöst und 18 Stunden bei 90° mit Phosphoroxychlorid (0,166 g) erhitzt wird. Abdampfen vom überschüssigen Phosphoroxychlorid mit Toluol und Zugabe bei 0° von gesättigter wässeriger Ammoniumhydroxidlösung, Extraktion der basischen Lösung mit Methylenchlorid (4 x 15 ml) und Trocknen des Extraktes über Natriumsulfat, ergibt ein Oel, welches chromatographiert (Silicagel, Essigsäureäthylester) wird. Man erhält das 6-(4-Methoxycarbonylbutyl)-imidazo[1,5-a]pyridin. Rf = 0,26; NMR ($CDCl_3$) 3,58 (s, 3H), 6,45 (d, 1H), 7,25 (d, 1H), 7,38 (s, 1H), 7,62 (s, 1H), 7,94 (s, 1H); IR (Methylenchlorid) 1730 $cm^{-1}$.

Der Ausgangsstoff wird wie folgt hergestellt:
2-Cyan-5-(4-methoxycarbonylbutyl)-pyridin (1,48 g, s. Beispiel 20) wird in 56 ml Methanol, welches 1,5 ml konz. Chlorwasserstoffsäure enthält, gelöst und über 0,75 g 10%-igem Palladium-auf-Kohle-Katalysator 18 Stunden bei atmosphärischem Druck hydriert. Das Gemisch wird filtriert, das Filtrat eingedampft, der Rückstand auf 20 g Silicagel chromatographiert und mit 1:1 Methanol-Essigsäureäthylester eluiert. Nach Umkristallisation aus Aether-Methylenchlorid erhält man das 2-Aminomethyl-5-(4-methoxycarbonylbutyl)-pyridin als sein Carbonat, welches bei 79-80° schmilzt. NMR ($CDCl_3$) 3,67 (s, 3H), 4,24 (s, 2H); IR (Methylenchlorid) 1725 $cm^{-1}$.

Beispiel 23: Eine Lösung von 92 mg 6-(4-Methoxycarbonylbutyl)-imidazo[1,5-a]pyridin in 0,3 ml Aethanol und 0,8 ml 1-normaler Natriumhydroxidlösung wird 2 Stunden unter Rückfluss sanft erhitzt, gekühlt, mit 2 ml Wasser verdünnt und mit Essigsäureäthylester (5 ml) extrahiert. Die wässerige Phase wird auf den pH-Wert 6 eingestellt und mit Chloroform extrahiert. Die Extrakte werden getrocknet und eingedampft. Man erhält das 6-(4-Carboxybutyl)-imidazo[1,5-a]pyridin, welches bei 168-171° schmilzt.

Beispiel 24: 2-(N-Formylaminomethyl)-4-(3-methoxycarbonylpropyl)-pyridin (33 mg) wird in 1 ml Toluol gelöst und mit Phosphoroxychlorid (44 mg) unter Stickstoff 18 Stunden bei 90° erhitzt. Das Lösungsmittel wird abgedampft, der Rückstand in Methylenchlorid suspendiert, auf 0° gekühlt und mit gesättigter wässeriger Ammoniumhydroxidlösung basisch gemacht. Die wässerige Phase wird mit Methylenchlorid (4 x 15 ml) extrahiert. Die Extrakte werden über Natriumsulfat getrocknet und eingedampft. Man erhält das 7-[3-Methoxycarbonylpropyl)-imidazo[1,5-a]pyridin als ein Oel, welches durch präparative Dünnschichtchromatographie gereinigt wird (Silicagel, 3:1 Essigsäureäthylester-Methanol). NMR ($CDCl_3$) 3,70 (s, 3H), 6,45 (q, 1H), 7,2 (s, 1H), 7,32 (s, 1H), 7,90 (d, 1H), 8,08 (s, 1H); IR (Methylenchlorid) 1730 $cm^{-1}$.

Der Ausgangsstoff wird wie folgt hergestellt:
Kaliumcyanid (11,18 g) und Dibenzo-18-Krone-6-äther (1,0 g) werden in einer Stickstoffatmosphäre zu einer Lösung von 4-(3-Chlorpropyl)-pyridin (6,68 g), (hergestellt aus 4-(3-Hydroxypropyl)-pyridin), in 300 ml trockenem Acetonitril gegeben. Das Gemisch wird 24 Stunden unter Rückfluss gekocht, das Lösungsmittel abgedampft und der Rückstand zwischen Methylenchlorid und Wasser verteilt. Die wässerige Phase wird mit Methylenchlorid (3 x 100 ml) weiter extrahiert. Die vereinigten Extrakte werden über Natriumsulfat getrocknet, mit Aktivkohle entfärbt und eingedampft. Man erhält das 4-(3-Cyanpropyl)-pyridin als ein farbloses Oel.

Durch eine Lösung von 4-(3-Cyanpropyl)-pyridin (5,5 g) in Methanol wird unter Eiskühlung Chlorwasserstoff 2 Stunden geleitet und die Lösung mit 100 ml Wasser vorsichtig versetzt. Die Lösung wird 15 Minuten gerührt und das Lösungsmittel abgedampft. Der Rückstand wird mit gesättigter wässeriger Natriumhydrogencarbonatlösung basisch gemacht und mit Methylenchlorid (3 x 100 ml) extrahiert. Der Extrakt wird über Natriumsulfat getrocknet und eingedampft. Der Rückstand wird in Aether durch 50 g Silicagel filtriert. Man erhält das 4-(3-Methoxycarbonylpropyl)-pyridin als ein Oel. NMR ($CDCl_3$) 3,68 (s, 3H), 7,05-7,25 (m, 2H), 8,45-8,65 (m, 2H); IR: 1725 $cm^{1}$.

Bei Zimmertemperatur gibt man Peressigsäure (40%, 2,9 ml) zu 4-(3-Methoxycarbonylpropyl)-pyridin (3,20 g). Das Gemisch wird bei 80° eine Stunde erhitzt und wenn der Test für Peroxid negativ ist, die

Essigsäure abgedampft. Der Rückstand wird in Methylenchlorid (50 ml) aufgenommen, filtriert und eingedampft. Das erhaltene 4-(3-Methoxycarbonylpropyl)-pyridin-N-oxid wird mit Dimethylsulfat (2,8 g, 22,2 mMol) in 12 ml Toluol 1 Stunde bei 80° behandelt. Nach Eindampfen des Lösungsmittels erhält man 5,45 g des 4-(3-Methoxycarbonylpropyl)-1-methoxypyridinium-methylsulfats, welches zu einer Lösung von 89,75 g Kaliumcyanid in 20 ml Wasser gegeben wird. Das Reaktionsgemisch wird eine Stunde bei 0° und 3 Stunden bei 25° gerührt und dann mit Methylenchlorid (1 x 30 ml) extrahiert. Die wässerige Phase wird 24 Stunden stehen gelassen und dann mit Methylenchlorid (1 x 30 ml) extrahiert. Die vereinigten Extrakte werden über Natriumsulfat getrocknet und eingedampft. Man erhält ein rotes Oel, welches nach Chromatographie auf 70 g Silicagel und Eluierung mit Aether, das 2-Cyan-4-(3-methoxycarbonylpropyl)-pyridin als ein Oel ergibt. NMR ($CDCl_3$) 3,67 (s, 3H), 7,42 (d, 1H), 7,60 (s, 1H), 8,60 (d, 1H); IR (Methylenchlorid) 1725 $cm^{-1}$.

2-Cyan-4-(3-methoxycarbonylpropyl)-pyridin (0,83 g) in 9 ml Methanol wird über 0,4 g 10%-igem Palladium-auf-Kohle-Katalysator 3 Stunden bei 3 Atmosphären hydriert. Das Gemisch wird filtriert, das Filtrat eingedampft und der Rückstand durch präparative Dünnschichtchromatographie auf Silicagel mit 1:1 Methanol-Essigsäureäthylester gereinigt. Man erhält das 2-Aminomethyl-4-(3-methoxycarbonylpropyl)-pyridin. Rf = 0,37 (Essigsäureäthylester-Methanol 1:1, 1% Ammoniumhydroxid); NMR ($CDCl_3$) 3,67 (s, 3H), 4,15 (s, 2H).

2-Aminomethyl-4-(3-methoxycarbonylpropyl)-pyridin (0,11 g) in 0,5 ml 97%-iger Ameisensäure wird 18 Stunden bei 90° erhitzt. Das Reaktionsgemisch wird auf Zimmertemperatur gekühlt, mit Ammoniumhydroxidlösung basisch gemacht und mit Methylenchlorid (4 x 20 ml) extrahiert. Die organischen Extrakte werden über Natriumsulfat getrocknet und eingedampft. Man erhält das 2-(N-Formylaminomethyl)-4-(3-methoxycarbonylpropyl)-pyridin. IR (Methylenchlorid) 1735, 1685 $cm^{-1}$.

Beispiel 25: 7-(3-Methoxycarbonylpropyl)-imidazo[1,5-a]-pyridin (Beispiel 24, 8,0 mg) wird in 0,3 Methanol gelöst und mit 0,1 ml 1-normaler Natriumhydroxidlösung versetzt. Das Gemisch wird 5 Stunden bei 25° gerührt, eingedampft und der Rückstand in 5 ml Wasser gelöst. Die wässerige Lösung wird mit 2 ml Essigsäureäthylester gewaschen, mit 2-normaler Schwefelsäure auf den pH-Wert 6 eingestellt und mit Methylenchlorid (3 x 5 ml) extrahiert. Die organischen Extrakte werden über Natriumsulfat/Magnesiumsulfat getrocknet und eingedampft. Man erhält das 7-(3-Carboxypropyl)-imidazo[1,5-a]pyridin; IR ($CHCl_3$) 1720 $cm^{-1}$.

Beispiel 26: Eine Lösung von 7-[4,4-(Bis-methoxycarbonyl)-butyl]-imidazo[1,5-a]pyridin (65 mg) in 0,8 ml 1-normaler Natriumhydroxydlösung und 0,5 ml Aethanol wird 2 Stunden unter Rückfluss erhitzt. Das Lösungsmittel wird abgedampft und der Rückstand mit 0,8 ml 1-normaler Chlorwasserstoffsäure versetzt. Das Wasser wird abgedampft, der Rückstand in 3 ml Xylol gelöst und 4 Stunden auf 137° erhitzt. Das Xylol wird abgedampft und durch 2 ml 1-normaler Natriumhydroxidlösung ersetzt. Die wässerige Phase wird mit Essigsäureäthylester (5 ml) extrahiert. Ansäuern auf den pH-Wert 6 und Re-extraktion mit Chloroform (3 x 15 ml) und Eindampfen ergibt das 7-(4-Carboxybutyl)-imidazo-[1,5-a]pyridin, welches bei 158-161° schmilzt.

Der Ausgangsstoff wird wie folgt hergestellt.
Gemäss den vorher beschriebenen Verfahren (z.B. Beispiele 20 und 24) wird das 4-(3-Chlorpropyl)-pyridin in das 4-(3-Chlorpropyl)-2-cyan-pyridin umgewandelt. NMR ($CDCl_3$) 3,56 (t, 2H), 7,40 (d, 1H), 7,57 (s, 1H), 8,60 (d, 1H).

Eine Lösung von Boran-dimethylsulfid (0,83 ml, 7,7 mMol) in 7 ml Tetrahydrofuran wird langsam zu einer unter Rückfluss kochenden Lösung von 4-(3-Chlorpropyl)-2-cyanpyridin (1,24 g, 6,9 mMol) in 7 ml Tetrahydrofuran gegeben, wobei gleichzeitig Dimethylsulfid abdestilliert

wird. Nach der abgeschlossenen Zugabe wird das Gemisch 15 Minuten unter Rückfluss gekocht, auf 30° gekühlt und mit 6-normaler Chlorwasserstoffsäure versetzt. Nach dem Aufhören der Wasserstoffentwicklung wird das Gemisch 30 Minuten unter Rückfluss gekocht, auf 0° gekühlt, mit festem Natriumcarbonat gesättigt und mit Methylenchlorid (4 x 50 ml) extrahiert. Die organischen Extrakte werden über Natriumsulfat getrocknet und eingedampft. Man erhält ein Oel, welches durch 10 g Silicagel (1:1 Essigsäureäthylester-Methanol) filtriert wird. Man erhält das 2-Aminomethyl-4-(3-chlorpropyl)-pyridin als ein gelbes Oel, NMR ($CDCl_3$) 3,55 (t, 2H), 4,20 (s, 2H).

Eine Lösung von 2-Aminomethyl-4-(3-chlorpropyl)-pyridin (0,47 g) in 1 ml Ameisensäure wird 18 Stunden bei 90° erhitzt, auf 0° gekühlt und mit gesättigter wässeriger Ammoniumhydroxidlösung basisch gemacht. Extraktion mit Methylenchlorid (4 x 10 ml), Trocknen über Magnesiumsulfat und Eindampfen ergibt das 2-(N-Formylaminomethyl)-4-(3-chlorpropyl)-pyridin (IR 1674 $cm^{-1}$). Dieses wird in Phophoroxychlorid (0,75 g) 15 Stunden bei 90° erhitzt. Das überschüssige Phosphoroxychlorid wird mit Toluol abgedampft, der Rückstand in Methylenchlorid (15 ml) suspendiert, auf 0° gekühlt und mit gesättigtem Ammoniumhydroxid basisch gemacht. Extraktion mit Methylenchlorid (4 x 15 ml), Trocknen über Natriumsulfat und Dünnschichtchromatographie (Silicagel, Essigsäureäthylester), des Rückstands ergibt das 7-(3-Chlorpropyl)-imidazo[1,5-a]pyridin (Rf = 0,24, Essigsäureäthylester) als ein gummiartiges Material. NMR ($CDCl_3$) 3,58 (t, 2H), 6,42 (q, 1H), 7,21 (s, 1H), 7,32 (s, 1H), 7,88 (d, 1H), 8,07 (s, 1H).

Eine Lösung von 7-(3-Chlorpropyl)-imidazo[1,5-a]pyridin (50 mg), Malonsäure-dimethylester (0,14 g) und Kaliumcarbonat (144 mg) in 2 ml Dimethylformamid wird unter Stickstoff 9 Stunden zwischen 80 und 90° erhitzt. Das Lösungsmittel wird eingedampft, der Rückstand in 10 ml Wasser aufgenommen und mit Essigsäureäthylester (2 x 10 ml) extrahiert. Die organischen Extrakte werden mit 2-normaler Chlorwasserstoffsäure

(2 x 10 ml) gewaschen. Die wässerigen Extrakte werden mit festem Natriumhydrogencarbonat basisch gemacht, mit Methylenchlorid (3 x 10 ml) extrahiert, über Natriumsulfat getrocknet und eingedampft. Man erhält das 7-[4,4-(Bis-methoxycarbonyl)-butyl]-imidazo[1,5-a]pyridin. NMR ($CDCl_3$) 3,40 (s, 6H), 6,06 (d, 1H); IR (Methylenchlorid) 1725 $cm^{-1}$.

Beispiel 27: 5-(4-Chlorbutyl)-imidazo[1,5-a]pyridin wird gemäss dem im Beispiel 4 für die Herstellung von 5-(5-Chlorpentyl)imidazo[1,5-a]-pyridin beschriebenen Verfahren, unter Verwendung von 1-Brom-3-chlor-propan als Reagens anstelle vom 1-Brom-4-chlorbutan hergestellt.

Beispiel 28: Die Umsetzung von 6-Benzyloxy-5-methylimidazo[1,5-a]-pyridin mit 5-Brom-1,1,1-triäthoxypentan wird gemäss dem in Beispiel 1 beschriebenen Verfahren durchgeführt, wobei man 6-Benzyloxy-5-(5-äthoxycarbonylpentyl)-imidazo[1,5-a]pyridin erhält. NMR ($CDCl_3$): 1,4 (3H), 4,17 (2H), 4,97 (2H).

Die Hydrolyse mit 1-n. wässriger Natronlauge und Aethanol (1:1) ergibt 6-Benzyloxy-5-(5-carboxypentyl)-imidazo[1,5-a]pyridin vom Smp. 139-141°.

Der Ausgangsstoff 6-Benzyloxy-5-methylimidazo[1,5-a]pyridin wird wie folgt hergestellt:

Eine Lösung von 8,54 g 3-Benzyloxy-6-hydroxymethyl-2-methylpyridin in 53 ml Thionylchlorid wird 2 Stunden unter Rückfluss gekocht und dann das Thionylchlorid durch Destillation entfernt. Der Rückstand wird auf 50 g Eis gegossen, mit gesättigter Natriumhydrogencarbonat-Lösung basisch gemacht und mit 3 x 50 ml Essigsäureäthylester extrahiert. Die organischen Extrakte werden über Natriumsulfat getrocknet, das Lösungsmittel abgedampft, und man erhält ein gelbes Oel, das als 3-Benzyloxy-6-chlormethyl-2-methylpyridin identifiziert wird; NMR ($CDCl_3$): 2,47 (3H), 4,54 (2H), 4,97 (2H).

Eine Lösung von 8,02 g 3-Benzyloxy-6-chlormethyl-2-methylpyridin und 5,98 g Natriumazid in 400 ml Aethanol wird 4 Stunden auf 80° erhitzt. Man lässt abkühlen, filtriert, verdampft das Lösungsmittel und erhält einen Rückstand, zu dem 150 ml 0,5-n. Natriumhydroxid-Lösung und 150 ml Aether gegeben werden. Die organische Phase wird über Natriumsulfat getrocknet, das Lösungsmittel abgedampft und man erhält 6-Azidomethyl-3-benzyloxy-2-methylpyridin; NMR ($CDCl_3$): 2,47 (3H), 4,27 (2H), 4,95 (2H).

1,27 g Lithiumaluminiumhydrid werden zu einer Lösung von 7,18 g 6-Azidomethyl-3-benzyloxy-2-methylpyridin in 120 ml trockenem Aether bei Raumtemperatur gegeben. Man rührt 45 Minuten, und gibt dann nacheinander zunächst 1,3 ml Wasser, dann 1,3 ml einer 15% Natriumhydroxid-Lösung und schliesslich 3,8 ml Wasser hinzu. Die Salze werden abfiltriert, das Filtrat eingedampft, und man erhält 6-Aminomethyl-3-benzyloxy-2-methylpyridin; NMR ($CDCl_3$): 1,67 (2H), 2,47 (3H), 3,8 (2H), 4,95 (2H).

Eine Lösung von 6,31 g 6-Aminomethyl-3-benzyloxy-2-methylpyridin in 8,7 ml Ameisensäure wird 15 Stunden auf 90° erhitzt. Man lässt abkühlen, macht mit eiskalter wässeriger Ammoniak-Lösung basisch und extrahiert mit 3 x 25 ml Chloroform. Die vereinigten Extrakte werden über Natriumsulfat getrocknet und das Lösungsmittel abgedampft. Man kristallisiert aus Aether und erhält 3-Benzyloxy-6-formylamino-methyl-2-methylpyridin vom Smp. 67-68°.

Eine Lösung von 5,14 g 3-Benzyloxy-6-formylaminomethyl-2-methylpyridin und 4,0 ml Phosphoroxychlorid in 18 ml Toluol wird 15 Stunden auf 90° erhitzt. Das Lösungsmittel wird abgedampft, der Rückstand in 50 ml Chloroform aufgenommen, auf 0° abgekühlt und mit eiskalter wässeriger Ammoniak-Lösung basisch gemacht. Die wässerigerPhase wird nochmals mit 3 x 20 ml Chloroform extrahiert und die Extrakte über Natriumsulfat getrocknet. Nach dem Abdampfen des Lösungsmittels

erhält man ein Oel, das, gelöst in Essigsäureäthylester, über 38 g Kieselgel geschickt wird. Man dampft das Lösungsmittel ab, kristallisiert den erhaltenen Feststoff aus Aether und erhält 6-Benzyloxy-5-methylimidazo[1,5-a]pyridin vom Smp. 52-54°.

Beispiel 29: Eine Mischung aus 4,0 g 5-(4-Cyanbutyl)-imidazo[1,5-a]pyridin, 1,88 g Natriumazid, 1,57 g Ammoniumchlorid und 4,24 g Lithiumchlorid in 14 ml trockenem Dimethylformamid wird 17 Stunden auf 125° erhitzt. Man kühlt ab, filtiert, verdampft das Lösungsmittel, löst das zurückbleibende Oel in 50 ml Wasser und extrahiert mit 50 ml Essigsäureäthylester. Die wässerige Phase wird auf pH 2 eingestellt, mit Essigsäureäthylester extrahiert und dann auf pH 5 gebracht. Der erhaltene Feststoff wird abfiltriert und in das entsprechende Hydrochlorid überführt, welches aus Aceton/Methanol kristallisiert wird. Man erhält 5-[4-(5-Tetrazolyl)butyl]-imidazo[1,5-a]pyridin-Monohydrochlorid vom Smp. 159-161°.

Das Ausgangsmaterial wird wie folgt hergestellt:

Eine Lösung von 5,73 g 5-(4-Chlorbutyl)imidazo[1,5-a]pyridin, 7,18 g Kaliumcyanid und 1,0 g Dibenzo-18-Krone-6-Aether in 190 ml Acetonitril wird 18 Stunden unter Rückfluss gekocht. Man kühlt ab, verdampft das Lösungsmittel, gibt zu dem zurückbleibenden Oel Wasser und Methylenchlorid und trennt die Phasen.Die organische Phase wird über Natriumsulfat getrocknet und das Lösungsmittel abgedampft. Der erhaltene Feststoff wird in Essigsäureäthylester gelöst und über 60 g Kieselgel filtriert, wobei man 5-(4-Cyanbutyl)-imidazo[1,5-a]pyridin vom Smp. 72-75° erhält.

Beispiel 30: Eine Lösung von 5,26 g 5-(5-Cyanpentyl)-imidazo[1,5-a]-pyridin in 16 ml trockenem Dimethylformamid wird mit 2,15 g Natriumazid, 0,2 g Lithiumchlorid und 1,80 g Ammoniumchlorid 15 Stunden auf 120° erhitzt. Man kühlt ab, filtriert, verdampft das Lösungsmittel und löst den Rückstand in 50 ml Wasser. Es wird mit 25 ml Essigsäureäthylester extrahiert, dann die wässerige Phase mit konz. Schwefelsäure auf pH 5 gebracht. Der ausgefallene Feststoff wird abfiltriert, mit Wasser gewaschen, getrocknet, und man erhält 5-[5-(5-Tetrazolyl)-pentyl]-imidazo[1,5-a]pyridin vom Smp. 149-150°. Das entsprechende Monohydrochlorid hat einen Smp. von 188-190°.

Der Ausgangsstoff, dessen Herstellung in Beispiel 4 beschrieben ist, kann auch wie folgt erhalten werden:

11,42 g Thionylchlorid werden bei Raumtemperatur langsam zu einer Suspension von 21,0 g 5-(5-Carboxypentyl)-imidazo[1,5-a]pyridin und 10,5 g Sulfamid[$SO_2(NH_2)_2$] in 90 ml Sulfolan gegeben. Das Gemisch wird auf 120° bis zum Ende der Gasentwicklung erhitzt, und dann vorsichtig 1,71 g p-Toluolsulfonsäure-Monohydrat hinzugegeben. Man erhitzt weitere 3 Stunden auf 120°, kühlt das Reaktionsgemisch ab, giesst es auf 200 g Eis und säuert es mit 130 ml 1-n. Salzsäure an. Die wässerige Phase wird dreimal mit je 100 ml Essigsäureäthylester extrahiert, sie wird dann mit festem Natriumhydrogencarbonat basisch gemacht und nochmals mit 3 x 125 ml Essigsäureäthylester extrahiert. Die organischen Extrakte werden mit 5 x 50 ml 0,5-n. Natronlauge gewaschen, über Natriumsulfat getrocknet und das Lösungsmittel verdampft, wobei man ein Oel erhält, das mit Essigsäureäthylester über 60 g Kieselgel chromatographiert wird. Man erhält 5-(5-Cyanpentyl)-imidazo[1,5-a]-pyridin als Oel.

Beispiel 31: Eine Hydroxylamin-Lösung, hergestellt aus 2,06 g Hydroxylamin-Hydrochlorid und 2,02 g Natriumhydroxid, und 6,08 g 5-(5-Methoxycarbonylpentyl)-imidazo[1,5-a]pyridin in 25 ml Methanol lässt man 20 Stunden bei Raumtemperatur stehen. Das Methanol wird verdampft, der Rückstand in 5 ml Wasser aufgenommen und auf pH 7 eingestellt. Das zunächst erhaltene Oel kristallisiert. Man sammelt den Feststoff, der 5-[5-(Hydroxycarbamoyl)pentyl]-imidazo[1,5-a]pyridin vom Smp. 138-140° ist.

Beispiel 32: 6,1 g Aethanolamin und 2,32 g 5-(5-Carboxypentyl)-imidazo[1,5-a]pyridin werden 3 Stunden auf 170° erhitzt. Ueberschüssiges Aethanolamin wird durch Destillation unter Vakuum entfernt, und man erhält 5-[5-(4,5-Dihydrooxazol-2-yl)pentyl)]-imidazo[1,5-a]pyridin.

Beispiel 33: Folgende Verbindungen der Formel I, worin B 5-Tetrazolyl oder Hydroxycarbamoyl bedeutet, können in analoger Weise zu den vorstehenden Beispielen hergestellt werden. "Position" gibt die Stellung an, in der die Gruppe $-CH_2-A-B$ an das Imidazo[1,5-a]-pyridin-Gerüst angeknüpft ist.

| Nr. | Position | A | $R_1$ | $R_2$ |
|---|---|---|---|---|
| 1 | 5 | $(CH_2)_4$ | 6-Benzyloxy | H |
| 2 | 5 | $(CH_2)_2CH=CH$ | H | H |
| 3 | 5 | $(CH_2)_4CH=CH$ | H | H |
| 4 | 8 | $(CH_2)_3$ | H | H |
| 5 | 6 | $(CH_2)_3$ | H | H |
| 6 | 7 | $(CH_2)_2$ | H | H |
| 7 | 7 | $(CH_2)_3$ | H | H |
| 8 | 5 | $(CH_2)_5$ | H | $3-CH_3$ |
| 9 | 5 | $(CH_2)_4$ | 6-Methoxy | H |

Der Ausgangsstoff für Verbindung 8, 3,5-Dimethylimidazo[1,5-a]pyridin, ist in J. Heterocyclic Chem. 3, 33 (1966) beschrieben.

Beispiel 34: Herstellung von 10'000 Tabletten mit einem Gehalt von je 10 mg der aktiven Substanz:

Bestandteile:

| | |
|---|---|
| 5-[4-(5-Tetrazolyl)butyl]-imidazo[1,5-a]pyridin | 100 g |
| Milchzucker | 1157 g |
| Maisstärke | 75 g |
| Polyäthylenglykol 6000 | 75 g |
| Talkpulver | 75 g |
| Magnesiumstearat | 18 g |
| gereinigtes Wasser | q.s. |

Verfahren: Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeingeten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 6,4 mm Durchmesser, welche eine Bruchrille aufweisen, gepresst.

Beispiel 35: Herstellung von 10'000 Kapseln mit einem Gehalt von je 25 mg der aktiven Substanz:

Bestandteile:

| | |
|---|---|
| 5-[5-(Hydroxycarbamoyl)pentyl]-imidazo[1,5-a]pyridin | 250 g |
| Milchzucker | 1650 g |
| Talkpulver | 100 g |

Verfahren: Sämtliche pulverigen Bestandteile werden mit einem Sieb von 0,6 mm Maschenweite gesiebt. Dann wird der Wirkstoff zuerst mit Talk und nachher mit Milchzucker in einem geeigneten Mischer homo-

genisiert. Kapseln Nr. 3 werden mit je 200 mg der erhaltenen Mischung in einer Füllmaschine abgefüllt.

In analoger Weise werden auch Tabletten und harte Gelatine-Kapseln unter Verwendung von 10-100 mg der Verbindungen der anderen Beispiele hergestellt.

Patentansprüche

(für alle Länder ausser Oesterreich)

1. Verbindungen der allgemeinen Formel I

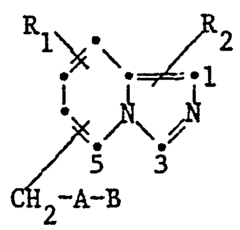

(I)

oder ihre 5,6,7,8-Tetrahydroderivate, worin $R_1$ für Wasserstoff, Halogen, Niederalkyl, Niederalkoxy, Hydroxy oder Aryl-niederalkoxy steht, $R_2$ Wasserstoff, Halogen oder Niederalkyl bedeutet, A für Alkylen mit 1 bis 12 Kohlenstoffatomen, Alkinylen oder Alkenylen mit 2 bis 12 Kohlenstoffatomen steht, und B 5-Tetrazolyl, Hydroxycarbamoyl oder unsubstituiertes oder durch Niederalkyl substituiertes 4,5-Dihydro-2-oxazolyl bedeutet, und ihre Salze.

2. Verbindungen der Formel I gemäss Anspruch 1, in welchen die Gruppe $-CH_2-A-B$ an die 5-Stellung gebunden ist, und ihre Salze.

3. Verbindungen der allgemeinen Formel II gemäss Anspruch 1,

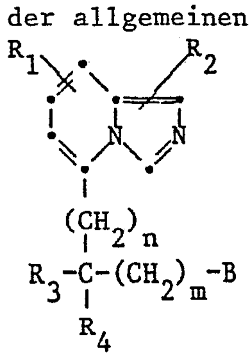

(II)

oder ihre 5,6,7,8-Tetrahydroderivate, worin jedes der Symbole $R_1$, $R_2$, $R_3$ und $R_4$ Wasserstoff oder Niederalkyl mit 1 bis 4 Kohlenstoffatomen bedeutet, n eine ganze Zahl von 1 bis 7 ist, m 0 oder 1 ist, B für 5-Tetrazolyl, Hydroxycarbamoyl oder unsubstituiertes oder durch Niederalkyl substituiertes 4,5-Dihydro-2-oxazolyl steht, und ihre Salze.

4. Verbindungen der im Anspruch 3 gezeigten Formel II gemäss Anspruch 1, in welchen die Gruppe

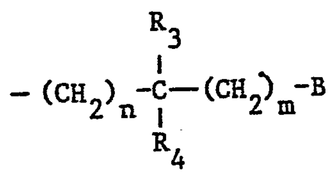

in der 5-Stellung angeknüpft ist, und ihre Salze.

5. Verbindungen der allgemeinen Formel III gemäss Anpruch 1,

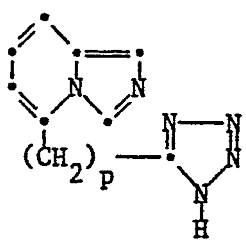

(III)

oder ihre 5,6,7,8-Tetrahydroderivate, worin p eine ganze Zahl von 3 bis 8 bedeutet, und ihre Salze.

6. Verbindungen der in Anspruch 5 gezeigten Formel III gemäss Anspruch 1 oder ihre 5,6,7,8-Tetrahydroderivate, worin p 4,5 oder 6 bedeutet, und ihre Salze.

7. 5-[4-(5-Tetrazolyl)butyl]-imidazo[1,5-a]pyridin und seine Salze.

8. 5-[5-(5-Tetrazolyl)pentyl]-imidazo[1,5-a]pyridin und seine Salze.

9. 5-[5-(Hydroxycarbamoyl)pentyl]-imidazo[1,5-a]pyridin und seine Salze.

10. Pharmazeutische Präparate enthaltend Verbindungen der Ansprüche 1 bis 9 oder ihre Salze, zusammen mit einem pharmazeutischen Trägermaterial.

11. Die in den Ansprüchen 1 bis 9 genannten Verbindungen zur Anwendung in einem Verfahren zur therapeutischen Behandlung des menschlichen oder tierischen Körpers.

12. Die in den Ansprüchen 1 bis 9 genannten Verbindungen als Hemmer der Thromboxan-Synthetase.

13. Verwendung der in den Ansprüchen 1 bis 9 genannten Verbindungen zur Herstellung von pharmazeutischen Präparaten.

14. Verfahren zur Herstellung von Verbindungen der allgemeinen Formel I gemäss Anspruch 1 und ihren Salzen, dadurch gekennzeichnet, dass man

A) in Verbindungen der Formel IV

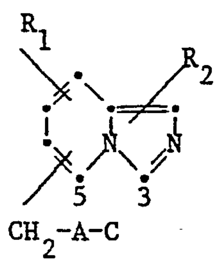

(IV)

oder ihren 5,6,7,8-Tetrahydroderivaten, worin $R_1$, $R_2$ und A die oben angegebene Bedeutung haben und C Carboxy, reaktionsfähiges

funktionell abgewandeltes Carboxy, Niederalkoxycarbonyl, Carbamoyl oder Cyan bedeutet, den Rest C in einen Rest B, wie unter der Formel I definiert, überführt, oder

B) eine Verbindung der Formel VI

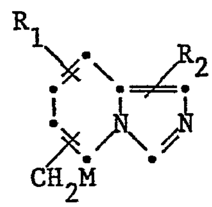

(VI),

worin M ein Alkalimetall bedeutet, $R_1$ Wasserstoff, Niederalkyl, Niederalkoxy oder Arylniederalkoxy ist und $R_2$ für Wasserstoff oder Niederalkyl steht, mit einem reaktionsfähigen funktionellen Derivat einer Verbindung der Formel VIIa

HO-A-B (VIIa),

worin A Alkylen mit 1 bis 12 Kohlenstoffatomen, Alkinylen oder Alkenylen mit 2 bis 12 Kohlenstoffatomen ist, und B 5-Tetrazolyl, Hydroxycarbamoyl oder unsubstituiertes oder durch Niederalkyl substituiertes 4,5-Dihydro-2-oxazolyl bedeutet, umsetzt, oder

C) eine Verbindung der Formel VIII

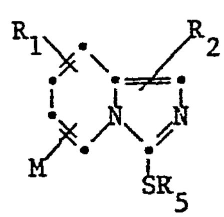

(VIII),

worin M ein Alkalimetall bedeutet, $R_1$ und $R_2$ die oben angegebene Bedeutung haben und $R_5$ Niederalkyl bedeutet, mit einem reaktionsfähigen funktionellen Derivat einer Verbindung der Formel IXa

$HOCH_2$-A-B (IXa),

worin A für Alkylen mit 1 bis 12 Kohlenstoffatomen, Alkinylen oder Alkenylen mit 2 bis 12 Kohlenstoffatomen steht, und B 5-Tetrazolyl, Hydroxycarbamoyl oder unsubstituiertes oder durch Niederalkyl substituiertes 4,5-Dihydro-2-oxazolyl bedeutet, umsetzt und die erhaltene Verbindung desulfuriert, oder

D) unter basischer Katalyse eine Verbindung der allgemeinen Formel X

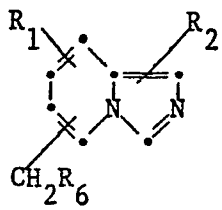

(X),

worin $R_1$ und $R_2$ die oben angegebene Bedeutung haben und $R_6$ für Niederalkoxycarbonyl oder Cyan steht, mit einem reaktionsfähigen funktionellen Derivat einer Verbindung der Formel VIIa

HO-A-B (VIIa),

worin A für Alkylen mit 1 bis 12 Kohlenstoffatomen, Alkinylen oder Alkenylen mit 2 bis 12 Kohlenstoffatomen steht, umsetzt, das Zwischenprodukt hydrolysiert und decarboxyliert, oder

E) eine Verbindung der Formel XIa

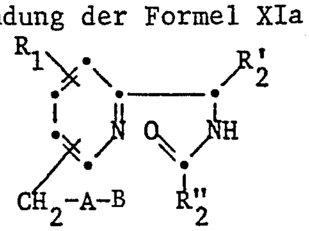

(XIa),

worin $R_1$ und A die oben angegebene Bedeutung haben, $R_2'$ und $R_2''$ Wasserstoff oder Niederalkyl bedeuten, A die oben angegebene Bedeutung hat, und B 5-Tetrazolyl, Hydroxycarbamoyl oder unsubstituiertes oder durch Niederalkyl substituiertes 4,5-Dihydro-2-oxazolyl bedeutet, ringschliesst, und, wenn erwünscht, eine erhaltene Verbindung der Formel I in eine andere Verbindung

der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

15. Die nach dem Verfahren des Anspruchs 14 erhältlichen Verbindungen.

16. Verbindungen der Formel IV, worin $R_1$ Hydroxy, Niederalkoxy oder Aryl-niederalkoxy ist, $R_2$ Wasserstoff oder Niederalkyl bedeutet, A für Alkylen mit 2 bis 12 Kohlenstoffatomen oder für Alkinylen oder Alkenylen mit 2 bis 12 Kohlenstoffatomen steht und C Carboxy, Niederalkoxycarbonyl, Carbamoyl oder Cyan bedeutet, und ihre Salze.

17. Verbindungen der Formel IV gemäss Anspruch 16, worin $R_1$ Hydroxy, Niederalkoxy oder Aryl-niederalkoxy ist, $R_2$ Wasserstoff oder Niederalkyl bedeutet, A für Alkylen mit 3 bis 8 Kohlenstoffatomen oder für Alkinylen oder Alkenylen mit 4 bis 8 Kohlenstoffatomen steht und C Carboxy bedeutet, und ihre Salze.

18. 6-Benzyloxy-5-(5-carboxypentyl)-imidazo[1,5-a]pyridin und seine Salze.

Patentansprüche

(für Oesterreich)

1. Verfahren zur Herstellung von neuen Imidazo[1,5-a]pyridinen der allgemeinen Formel I

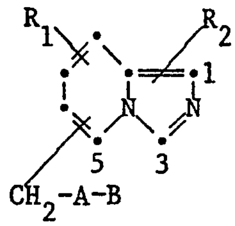

(I)

oder ihren 5,6,7,8-Tetrahydroderivaten, worin $R_1$ für Wasserstoff, Halogen, Niederalkyl, Niederalkoxy, Hydroxy oder Aryl-niederalkoxy steht, $R_2$ Wasserstoff, Halogen oder Niederalkyl bedeutet, A für Alkylen mit 1 bis 12 Kohlenstoffatomen, Alkinylen oder Alkenylen mit 2 bis 12 Kohlenstoffatomen steht, und B 5-Tetrazolyl, Hydroxycarbamoyl oder unsubstituiertes oder durch Niederalkyl substituiertes 4,5-Dihydro-2-oxazolyl bedeutet, und ihren Salzen, dadurch gekennzeichnet, dass man

A) in Verbindungen der Formel IV

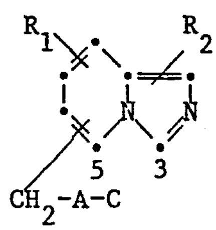

(IV)

oder ihren 5,6,7,8-Tetrahydroderivaten, worin $R_1$, $R_2$ und A die oben angegebene Bedeutung haben und C Carboxy, reaktionsfähiges funktionell abgewandeltes Carboxy, Niederalkoxycarbonyl, Carbamoyl oder Cyan bedeutet, den Rest C in einen Rest B, wie unter der Formel I definiert, überführt, oder

B) eine Verbindung der Formel VI

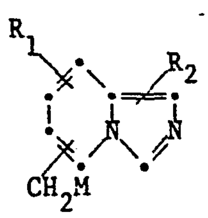

(VI),

worin M ein Alkalimetall bedeutet, $R_1$ Wasserstoff, Niederalkyl, Niederalkoxy oder Arylniederalkoxy ist und $R_2$ für Wasserstoff oder Niederalkyl steht, mit einem reaktionsfähigen funktionellen Derivat einer Verbindung der Formel VIIa

HO-A-B (VIIa),

worin A Alkylen mit 1 bis 12 Kohlenstoffatomen, Alkinylen oder Alkenylen mit 2 bis 12 Kohlenstoffatomen ist, und B 5-Tetrazolyl, Hydroxycarbamoyl oder unsubstituiertes oder durch Niederalkyl substituiertes 4,5-Dihydro-2-oxazolyl bedeutet, umsetzt, oder

C) eine Verbindung der Formel VIII

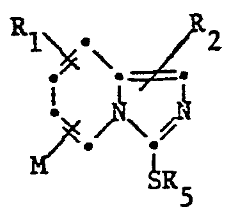

(VIII),

worin M ein Alkalimetall bedeutet, $R_1$ und $R_2$ die oben angegebene Bedeutung haben und $R_5$ Niederalkyl bedeutet, mit einem reaktionsfähigen funktionellen Derivat einer Verbindung der Formel IXa

$HOCH_2$-A-B (IXa),

worin A für Alkylen mit 1 bis 12 Kohlenstoffatomen, Alkinylen oder Alkenylen mit 2 bis 12 Kohlenstoffatomen steht, und B 5-Tetrazolyl, Hydroxycarbamoyl oder unsubstituiertes oder durch Niederalkyl substituiertes 4,5-Dihydro-2-oxazolyl bedeutet, umsetzt und die erhaltene Verbindung desulfuriert, oder

D) unter basischer Katalyse eine Verbindung der allgemeinen Formel X

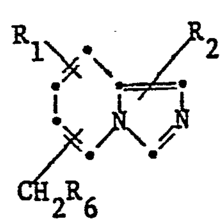

(X),

worin $R_1$ und $R_2$ die oben angegebene Bedeutung haben und $R_6$ für Niederalkoxycarbonyl oder Cyan steht, mit einem reaktionsfähigen funktionellen Derivat einer Verbindung der Formel VIIa

HO-A-B (VIIa),

worin A für Alkylen mit 1 bis 12 Kohlenstoffatomen, Alkinylen oder Alkenylen mit 2 bis 12 Kohlenstoffatomen steht, umsetzt, das Zwischenprodukt hydrolysiert und decarboxyliert, oder

E) eine Verbindung der Formel XIa

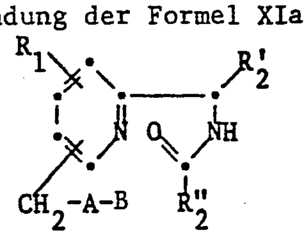

(XIa),

worin $R_1$ und A die oben angegebene Bedeutung haben, $R_2'$ und $R_2''$ Wasserstoff oder Niederalkyl bedeuten, A die oben angegebene Bedeutung hat, und B 5-Tetrazolyl, Hydroxycarbamoyl oder unsubstituiertes oder durch Niederalkyl substituiertes 4,5-Dihydro-2-oxazolyl bedeutet, ringschliesst, und, wenn erwünscht, eine erhaltene Verbindung der Formel I in eine andere Verbindung der Erfindung umwandelt, und/oder, wenn erwünscht, eine erhaltene freie Verbindung in ein Salz oder ein erhaltenes Salz in die freie Verbindung oder in ein anderes Salz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

2. Verfahren gemäss Anspruch 1, dadurch gekennzeichnet, dass man Verbindungen der im Anspruch 1 gezeigten Formel I, in welchen die Gruppe $-CH_2-A-B$ an die 5-Stellung gebunden ist, und ihre Salze herstellt.

3. Verfahren gemäss Anspruch 1, dadurch gekennzeichnet, dass man Verbindungen der allgemeinen Formel II

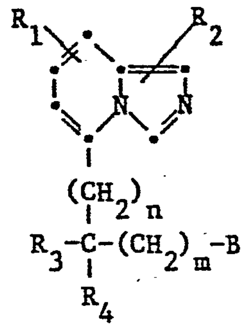

(II)

oder ihre 5,6,7,8-Tetrahydroderivate, worin jedes der Symbole $R_1$, $R_2$, $R_3$ und $R_4$ Wasserstoff oder Niederalkyl mit 1 bis 4 Kohlenstoffatomen bedeutet, n eine ganze Zahl von 1 bis 7 ist, m 0 oder 1 ist, B für 5-Tetrazolyl, Hydroxycarbamoyl oder unsubstituiertes oder durch Niederalkyl substituiertes 4,5-Dihydro-2-oxazolyl steht, und ihre Salze herstellt.

4. Verfahren gemäss Anspruch 1, dadurch gekennzeichnet, dass man Verbindungen der im Anspruch 3 gezeigten Formel II, in welchen die Gruppe

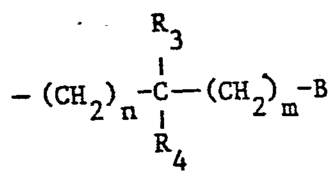

in der 5-Stellung angeknüpft ist, und ihre Salze herstellt.

5. Verfahren gemäss Anspruch 1, dadurch gekennzeichnet, dass man Verbindungen der allgemeinen Formel III

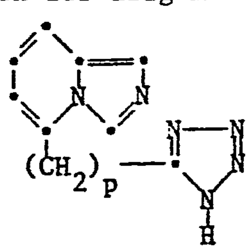

(III)

oder ihre 5,6,7,8-Tetrahydroderivate, worin p eine ganze Zahl von 3 bis 8 bedeutet, und ihre Salze herstellt.

6. Verfahren gemäss Anspruch 1, dadurch gekennzeichnet, dass man Verbindungen der im Anspruch 5 gezeigten Formel III oder ihre 5,6,7,8-Tetrahydroderivate, worin p 4,5 oder 6 bedeutet, und ihre Salze herstellt.

7. Verfahren gemäss Anspruch 1, dadurch gekennzeichnet, dass man 5-[4-(5-Tetrazolyl)butyl]-imidazo[1,5-a]pyridin und seine Salze herstellt.

8. Verfahren gemäss Anspruch 1, dadurch gekennzeichnet, dass man 5-[5-(5-Tetrazolyl)pentyl]-imidazo[1,5-a]pyridin und seine Salze herstellt.

9. Verfahren gemäss Anspruch 1, dadurch gekennzeichnet, dass man 5-[5-(Hydroxycarbamoyl)pentyl]-imidazo[1,5-a]pyridin und seine Salze herstellt.

10. Verfahren zur Herstellung eines pharmazeutischen Präparates, gekennzeichnet durch die Verarbeitung eines erfindungsgemässen Wirkstoffes nach einem der Ansprüche 1 bis 9 mit einem pharmazeutischen Trägermaterial.

Office européen des brevets

# RAPPORT DE RECHERCHE EUROPEENNE

0114572
Numéro de la demande

EP 83 81 0595

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | FR-A-2 130 215 (KYORIN SEIYAKU)<br>* Patentansprüche *<br>--- | 1-11, 13,16-18 | C 07 D 471/04<br>A 61 K 31/44 //<br>C 07 D 213/36<br>C 07 D 213/54<br>C 07 D 213/65<br>C 07 D 213/84<br>C 07 D 213/89<br>(C 07 D 471/04<br>C 07 D 235/00<br>C 07 D 221/00 ) |
| A,D | TETRAHEDRON LETTERS, Band21, Nr. 22, 1980, Pergamon Press Ltd., (GB)<br>Ph. BLATCHER et al.: "A direct method for the substitution of imidazo (1,5-a) pyridines at position 5", Seiten 2195-2196. * ganzes Dokument *<br>--- | 1-9,14-18 | |
| P | EP-A-0 068 386 (CIBA-GEIGY)<br>* Patentansprüche *<br>----- | 1-18 | |

| DOMAINES TECHNIQUES RECHERCHES (Int. Cl. 3) |
|---|
| C 07 D 471/00<br>A 61 K 31/00 |

Le présent rapport de recherche a été établi pour toutes les revendications

| Lieu de la recherche | Date d'achèvement de la recherche | Examinateur |
|---|---|---|
| DEN HAAG | 29-03-1984 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82